# FASCICULE DE BREVET EUROPEEN

(11) **EP 1 328 546 B1**
(45) Date de publication et mention de la délivrance du brevet: **21.02.2007**
(21) Numéro de dépôt: 01974427.5
(22) Date de dépôt: 02.10.2001
(51) Int. Cl.: C07K 14/52, C07K 17/14, C07K 1/10, C07K 7/64, A61K 38/10, A61P 9/00

(54) **CYCLOPEPTIDES, LEUR PROCEDE DE PREPARATION ET LEUR UTILISATION COMME INHIBITEUR OU ACTIVATEUR DE L'ANGIOGENESE**
CYCLOPEPTIDE, VERFAHREN ZU IHRER HERSTELLUNG UND IHRE VERWENDUNG ZUR HEMMUNG ODER STIMULIERUNG VON ANGIOGENESE
CYCLIC PEPTIDES, METHOD FOR PREPARING SAME AND USE AS ANGIOGENESIS INHIBITOR OR ACTIVATOR

(30) Priorité: 04.10.2000 FR 0012654
(43) Date de publication de la demande: 23.07.2003
(73) Titulaire: COMMISSARIAT A L'ENERGIE ATOMIQUE, 75015 Paris (FR); Université Victor Segalen Bordeaux 2, 33076 Bordeaux Cedex (FR); Université Bordeaux 1, 33405 Talence Cedex (FR)
(72) Inventeur: BETZ, Natacha, F-92410 Ville d'Avray (FR); BIKFALVI, Andréas, F-33170 Gradignan (FR); DELERIS, Gérard, F-33000 Bordeaux (FR)
(74) Mandataire: Poulin, Gérard
(86) Numéro de dépôt international: PCT/FR2001/003049
(87) Numéro de publication internationale: WO 2002/028895

(56) Documents cités:
- EP-A- 0 844 252
- WO-A-00/53219
- WO-A-99/40947
- US-A- 5 849 692
- IVANOV B ET AL: "SYNTHESIS AND USE OF A NEW BROMOACETYL-DERIVATIZED HETEROTRIFUNCTIONAL AMINO ACID FOR CONJUGATION OF CYCLIC RGD-CONTAINING PEPTIDES DERIVED FROM HUMAN BONE SIALOPROTEIN" BIOCONJUGATE CHEMISTRY, AMERICAN CHEMICAL SOCIETY, WASHINGTON, US, vol. 6, 1995, pages 269-277, XP002063396 ISSN: 1043-1802
- DELFORGE D ET AL: "SOLID-PHASE SYNTHESIS OF TAILED CYCLIC PEPTIDES: THE USE OF ALPHA-ALLYL-PROTECTED ASPARTIC ACID LEADS TO ASPARTIMIDE AND TETRAMETHYLGUANIDINIUM FORMATION" LETTERS IN PEPTIDE SCIENCE, ESCOM SCIENCE PUBLISHERS, NL, vol. 3, 1 mai 1996 (1996-05-01), pages 89-97, XP002063392 ISSN: 0929-5666
- KATES S A ET AL: "AUTOMATED ALLYL CLEAVAGE FOR CONTINUOUS-FLOW SYNTHESIS OF CYCLIC AND BRANCHED PEPTIDES" ANALYTICAL BIOCHEMISTRY, ACADEMIC PRESS, SAN DIEGO, CA, US, vol. 212, 1993, pages 303-310, XP002063395 ISSN: 0003-2697

## Description

### Domaine technique

La présente invention a pour objet de nouveaux cyclopeptides et des systèmes les comportant qui permettent de maîtriser l'angiogenèse.

L'angiogenèse est un mécanisme de néo-vascularisation prenant naissance à partir d'un réseau capillaire préexistant. Elle est particulièrement importante et indispensable au cours de nombreux processus physiologique tels que le développement embryonnaire, l'implantation du placenta, mais aussi dans différentes pathologies, en particulier la croissance des tumeurs, le développement des métastases, l'ischémie, les maladies vasculaires de l'oeil et les maladies inflammatoires chroniques (voir Ferrara et al, Nature Medicine, vol. 5, n°12, Décembre 1999, pages 1361-1364 [1] et Hagedorn et Bikfalvi [2]). L'angiogenèse est aussi essentielle dans la régénération tissulaire et la colonisation durable des biomatériaux implantés tels que les substituts osseux.

L'angiogenèse est un processus multiphasique qui fait appel dans un premier temps à la migration, l'attachement et l'adhésion des cellules endothéliales, puis à leur prolifération et leur organisation en tubes, afin de former le réseau vasculaire nécessaire au développement des tissus.

Parmi les facteurs régulateurs de l'angiogenèse, le facteur de croissance de l'endothélium vasculaire (VEGF) est apparu comme l'un des plus importants.

Le VEGF existe sous quatre isoformes, A, B, C et D ; et parmi celles-ci, l'isoforme A qui comprend 165 aminoacides, est un régulateur puissant de l'angiogenèse tumorale et semble être impliqué dans d'autres pathologies comme la rétinopathie diabétique ou les maladies inflammatoires chroniques.

Le VEGF-A est produit par des cellules normales ou transformées. Son expression peut être induite par l'hypoxie, l'activation oncogénique ou l'activation par des facteurs de croissance, comme le facteur de croissance des fibroblastes FGF-2.

Le VEGF-A se fixe sur différents récepteurs, notamment le récepteur à domaine kinase KDR (VEGFR 2), qui semble être un effecteur très important dans l'angiogenèse pathologique. Aussi, l'inhibition de l'angiogenèse à travers le récepteur KDR pourrait constituer une approche thérapeutique intéressante.

La structure du VEGF-A qui comprend 165 aminoacides, a été décrite à la fin de 1997, et accessible à la fin juin 1998, comme il ressort du document (voir Muller Y. A. dans Structure, 1997, 5, pages 1325-1338, [3]).

### Etat de la technique antérieure

Un certain nombre de stratégies ont été développées dans le but d'interférer avec la fonction du récepteur KDR de VEGF. Elles incluent l'inhibition du VEGF
- par des anticorps humanisés comme il est décrit par Presta et al dans Cancer Research, 57 1997, pages 4593-4599 [4],
- par des anticorps antiidiotypes, comme il est décrit par Ortéga et al dans Am. J. Pathol. Nov. 1997, 151 (5), pages 1215-1224 [5],
- par des inhibiteurs du domaine tyrosine kinase du récepteur KDR comme il est décrit par Piossek et al dans The Journal of Biological Chemistry, vol. 274, n°9, 1999, pages 5612-5619 [6], et
- par des peptides inhibiteurs isolés par display de phage, comme il est décrit par Fairbrother et al dans Biochemistry 37, 1998, pages 17754-17764 [7].

D'autres molécules actives vis-à-vis de l'angiogenèse ont été caractérisées et certaines sont entrées en phase clinique en cancérologie, comme il est décrit par Hagedorn et Bikfalvi dans Critical Reviews in Oncology/Hematology, 34, 2000, pages 89-110 [2].

Le document US-A-5 939 383 [8] envisage l'utilisation de divers cyclopeptides greffés ou couplés à un support solide ou autre, pour des applications biotechnologiques. Parmi diverses possibilités, il propose le cyclopeptide suivant :
cyclo(Glu-Gln-Ile-Met-Arg-Ile-Lys-Pro-His-Gln) (SEQ ID NO : 24) pour le récepteur KDR du VEGF.

Cependant, il ne donne aucun résultat sur l'inhibition possible du récepteur KDR par ce cyclopeptide et, comme on le verra plus loin, celui-ci ne conduit pas à l'inhibition de la liaison du VEGF à son récepteur.

### Exposé de l'invention

La présente invention a précisément pour objet de nouveaux cyclopeptides ayant une affinité de liaison avec le récepteur KDR plus élevée que celle fournie par les produits mentionnés ci-dessus, ce qui permet leur utilisation dans des systèmes activateurs ou inhibiteurs de l'angiogenèse.

La présence des trois aminoacides Arg, Lys et His est essentielle pour obtenir une interaction voulue avec le récepteur KDR du VEGF.

Dans l'invention, les résidus Arg et Gly du cyclopeptide sont réunis par une chaîne qui peut comporter une ou plusieurs molécules organiques choisies parmi les acides aminés naturels et synthétiques, soit des composés comportant un groupe COOH et un groupe NH₂ éventuellement substitué. Les acides aminés peuvent être sous forme L ou sous forme D.

Les acides aminés synthétiques peuvent être par exemple des composés aromatiques et/ou hétérocycliques comportant un groupe COOH et un groupe NH2 sur une structure permettant de donner une conformation spatiale proche de celle du VEGF à la séquence peptidique intéressante (SEQ ID NO : 1).

Les parties aromatiques peuvent être dérivées du benzène, du naphtalène, du dibenzofurane.

Les hétéroatomes peuvent être des atomes d'oxygène, d'azote, de silicium, de germanium, d'étain ou de phosphore.

A titre d'exemple d'un tel acide aminé, on peut citer l'acide 4-(2' aminoéthyl)-6-dibenzofuranepropanoïque de formule :

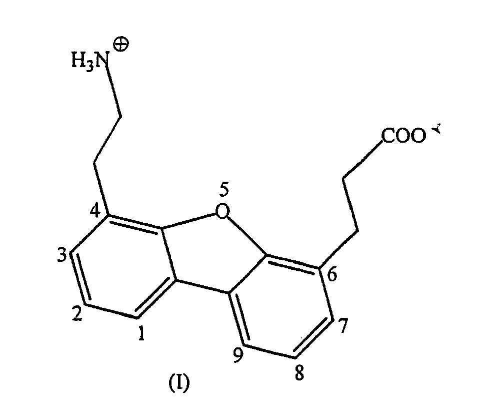

La synthèse de cet acide a été décrite par Bekele et al, J. Org. Chem. 62, 1997, pages 2259-2262 [9].

A titre d'exemple de composés organiques susceptibles d'être utilisés, on peut citer les sila-xanthènes, l'acide 4-(2'-aminoéthyl)-6-(dibenzofuranpropanoïque, et l'acide 5-(2'-aminoéthyl)-9,9-diméthylsila-xanthène-4-propanoïque.

Lorsque le composé comporte un groupe NH₂ substitué, celui-ci peut être du type NHR avec R représentant un groupe hydrocarboné, comportant éventuellement des groupes fonctionnels du type carboxy, ester, éther, hydroxy et siloxy.

De préférence, la chaîne qui lie les extrémités de la séquence peptidique. comprend un acide aminé sous forme D, de préférence D-Phe ou D-Tyr.

A titre d'exemple de cyclopeptides conformes à l'invention, on peut citer les cyclopeptides suivants :
P11 : cyclo(DPhe-Pro-Gln-Ile-Met-Arg-Ile-Lys-Pro-His-Gln-Gly-Gln-His-Ile-Gly-Glu) SEQ ID NO : 5
P16 : cyclo(Arg-Ile-Lys-Pro-His-Gln-Gly) SEQ ID NO : 8
P17 : cyclo(Pro-Arg-Ile-Lys-Pro-His-Gln-Gly) SEQ ID NO : 9
P19 : cyclo(Gln-Ile-Met-Arg-Ile-Lys-Pro-His-Gln-Gly-Gln-His-Ile-Gly-Glu) SEQ ID NO: 10
P20 : cyclo(DPhe-Pro-Gln-Ile-Met-Arg-Ile-Lys-Pro-His-Gln-Gly-Gln-His-Ile-Gly) SEQ ID NO :11
P23 : cyclo(DPhe-Pro-Arg-Ile-Lys-Pro-His-Gln) SEQ ID NO :13
P24 : cyclo(Gly-Arg-Ile-Lys-Pro-His) SEQ ID NO : 25

Les cyclopeptides de l'invention peuvent être utilisés pour maîtriser l'angiogenèse et traiter les diverses pathologies liées à l'angiogenèse. Pour ces applications, on peut les utiliser sous forme de solution ou dans des systèmes ou biomatériaux.

Dans le cas où les cyclopeptides sont utilisés sous forme de solution, il s'agit généralement de solutions aqueuses administrables par voie orale ou injectables. Le cyclopeptide peut être utilisé soit pour assurer l'inhibition de l'angiogenèse par fixation du cyclopeptide sur un récepteur VEGF, soit pour assurer l'activation de l'angiogenèse en couplant deux cyclopeptides sur un composé organique approprié pour leur donner une configuration spatiale efficace, autorisant la dimérisation des récepteurs du VEGF.

Dans les deux cas, les cyclopeptides peuvent être couplés si nécessaire à des agents bioactifs.

Aussi, l'invention a également pour objet une composition comprenant un cyclopeptide choisi parmi les cyclopeptides :
P11 : cyclo(DPhe-Pro-Gln-Ile-Met-Arg-Ile-Lys-Pro-His-Gln-Gly-Gln-His-Ile-Gly-Glu) SEQ ID NO : 5
P16 : cyclo(Arg-Ile-Lys-Pro-His-Gln-Gly) SEQ ID NO : 8
P17 : cyclo(Pro-Arg-Ile-Lys-Pro-His-Gln-Gly) SEQ ID NO : 9
P19 : cyclo(Gln-Ile-Met-Arg-Ile-Lys-Pro-His-Gln-Gly-Gln-His-Ile-Gly-Glu) SEQ ID NO :10
P20 : cyclo(DPhe-Pro-Gln-Ile-Met-Arg-Ile-Lys-Pro-His-Gln-Gly-Gln-His-Ile-Gly) SEQ ID NO :11
P23 : cyclo(DPhe-Pro-Arg-Ile-Lys-Pro-His-Gln) SEQ ID NO :13
P24 : cyclo(Gly-Arg-Ile-Lys-Pro-His) SEQ ID NO : 25

L'invention a encore pour objet une composition pharmaceutique pour activer l'angiogenèse comprenant deux cyclopeptides identiques ou différents, couplés avec un composé organique pharmaceutiquement acceptable, les cyclopeptides étant choisis parmi les cyclopeptides :
P11 : cyclo(DPhe-Pro-Gln-Ile-Met-Arg-Ile-Lys-Pro-His-Gln-Gly-Gln-His-Ile-Gly-Glu) SEQ ID NO : 5
P16 : cyclo(Arg-Ile-Lys-Pro-His-Gln-Gly) SEQ ID NO : 8
P17 : cyclo(Pro-Arg-Ile-Lys-Pro-His-Gln-Gly) SEQ ID NO : 9
P19 : cyclo(Gln-Ile-Met-Arg-Ile-Lys-Pro-His-Gln-Gly-Gln-His-Ile-Gly-Glu) SEQ ID NO:10
P20 : cyclo(DPhe-Pro-Gln-Ile-Met-Arg-Ile-Lys-Pro-His-Gln-Gly-Gln-His-Ile-Gly) SEQ ID NO :11
P23 : cyclo(DPhe-Pro-Arg-lle-Lys-Pro-His-Gln) SEQ ID NO :13
P24 : cyclo(Gly-Arg-Ile-Lys-Pro-His) SEQ ID NO : 25

Le composé organique pharmaceutiquement acceptable peut comporter des séquences alternées hydrophiles-hydrophobes et des fonctions réactives avec les fonctions des chaînes latérales des acides aminés du cyclopeptide.

Lorsque les cyclopeptides sont utilisés sous forme de systèmes ou biomatériaux, ceux-ci peuvent également être réalisés de façon à assurer, soit l'inhibition, soit l'activation de l'angiogenèse.

Selon un premier mode de réalisation de ces systèmes, ceux-ci comportent un ou plusieurs cyclopeptides, lié(s) chacun de façon covalente à un bras espaceur organique qui peut être lui-même lié de façon covalente à un support.

Avec le premier mode de réalisation, lorsque le système est en contact avec les récepteurs du VEGF, un seul cyclopeptide se fixe sur un récepteur du VEGF et empêche la dimérisation du récepteur et l'activation de l'angiogenèse.

Selon un seconde mode de réalisation de ces systèmes, plus particulièrement destiné à assurer, l'activation de l'angiogenèse, ceux-ci comportent deux cyclopeptides liés de façon covalente à un composé organique, pour disposer les deux cyclopeptides dans une configuration spatiale efficace pour activer l'angiogenèse.

Les composés organiques sont des composés pharmaceutiquement acceptables. Ils peuvent être formés de séquences alternées hydrophiles-hydrophobes, par exemple du type polyéthylène glycol/alcane ou fluoroalcane, et comporter des fonctions réactives avec les fonctions amines, hydroxy, acides carboxyliques ou autres des chaînes latérales du cyclopeptide pour assurer sa fixation sur le composé ; ils peuvent aussi comporter des fonctions de type acrylique.

A titre d'exemple de composé de ce type, on peut citer le composé de formule :

HOOCH₂CH₂O(CH₂CH₂O)₅(CH₂)₃-NH- (CH₂CH₂O)5CH₂-CH₂-COOH

qui comporte deux fonctions carboxyliques susceptibles de réagir avec des fonctions hydroxy ou amino des chaînes latérales des acides aminés du cyclopeptide

Les composés organiques susceptibles d'être utilisés peuvent aussi comporter des parties aromatiques et/ou des hétéroatomes de façon à maintenir, d'une part, les deux cyclopeptides dans une configuration efficace et d'autoriser, d'autre part, la fixation de ces deux cyclopeptides, dans cette configuration, sur divers supports.

Les parties aromatiques peuvent être dérivées du benzène, du naphtalène, du dibenzofurane.

Les hétéroatomes peuvent être des atomes d'oxygène, d'azote, de silicium, de germanium, d'étain ou de phosphore.

Pour assurer l'activation de l'angiogenèse, la distance entre les deux cyclopeptides fixés sur le composé organique est telle que, lorsque le système est mis en contact avec des cellules exprimant les récepteurs du facteur de croissance de l'endothélium vasculaire VEGF, il autorise la dimérisation de ces récepteurs.

Dans ce second mode de réalisation, le composé organique supportant les deux cyclopeptides est généralement relié à un support au moyen d'un bras espaceur organique.

Dans les deux modes de réalisation des systèmes de l'invention, le bras espaceur comprend par exemple une chaîne hydrocarbonée, fluorocarbonée, polyéther, polyéthylène glycol, polyamine, polyamide, polyester, polysiloxane ou une combinaison de celles-ci, qui est fonctionnalisée à chaque extrémité pour former une liaison covalente, d'une part, avec le cyclopeptide et, d'autre part, avec le support.

De préférence, le bras espaceur organique comprend de plus un motif ou une séquence peptitidique susceptible d'être coupé par un système enzymatique.

La coupure du bras espaceur permet ainsi de libérer les cyclopeptides actifs aux endroits voulus. Le système enzymatique peut être constitué par des métalloprotéases de la matrice extracellulaire ou d'autres enzymes.

Dans ce cas, le motif est une séquence peptidique qui est un substrat de ces métalloprotéases de la matrice extracellulaire, par exemple la séquence peptidique suivante :
HOOC-Ala-Gly-Leu-Leu-Gly-Gln-Pro-Gly-NH₂

Selon une disposition avantageuse de l'invention, le bras espaceur peut comprendre en outre des composés bioactifs qui seront également libérés à l'endroit voulu lors de la coupure de ce bras espaceur.

Ces composés bioactifs peuvent être par exemple des agents cytotoxiques, des agents anticancéreux ou tout autre principe actif que l'on voudrait amener au niveau des récepteurs KDR.

Selon l'invention, le support sur lequel sont fixés le ou les cyclopeptides peut être un solide organique ou inorganique. On peut utiliser en particulier, comme support un polymère organique sous forme solide ou sous forme de gel.

Le polymère utilisé est avantageusement un polymère biocompatible, biodégradable ou non biodégradable.

A titre d'exemple de polymères susceptibles d'être utilisés, on peut citer le polytéréphtalate d'éthylène, les copolymères de fluorure de vinylidène et d'hexafluoropropylène, les alcools polyvinyliques, les polyhydroxyéthyl méthacrylate, les polysaccharides et les copolymères obtenus à partir des monomères entrant dans la constitution des polymères ci-dessus.

Les cyclopeptides de l'invention peuvent être préparés par des procédés mettant en jeu une étape de synthèse automatique d'un peptide linéaire sur une phase solide par un procédé classique, suivie d'un couplage des extrémités du peptide linéaire, soit après avoir libéré le peptide de la phase solide, soit en le libérant ensuite de la phase solide.

Ainsi, selon un premier mode de réalisation, le procédé consiste
a) à préparer un peptide linéaire par synthèse chimique sur une phase solide,
b) à libérer le peptide linéaire de la phase solide, et
c) à coupler les extrémités du peptide linéaire pour former le cyclopeptide.

Selon un second mode de réalisation, le procédé consiste :
a) à préparer un peptide linéaire par synthèse chimique sur une phase solide,
b) à coupler l'extrémité libre du peptide linéaire avec une fonction terminale d'un résidu d'acide aminé du peptide linéaire, et
c) à libérer le cyclopeptide de la phase solide.

L'invention a encore pour objet un procédé de préparation d'un système comportant un support sur lequel sont fixés le(s) cyclopeptide(s), qui consiste à soumettre un support en polymère organique à une irradiation au moyen de rayonnements ionisants, de plasmas ou de photons, sur des zones définies du support, et à greffer ensuite sur ces zones du support un bras espaceur organique sur lequel est ou sera fixé le cyclopeptide.

Généralement, on réalise l'irradiation au travers d'un masque pour définir les zones du support à modifier. Les rayonnements ionisants utilisés peuvent être des faisceaux d'électrons ou d'ions lourds rapides.

Dans ce procédé, les cyclopeptides peuvent être fixés sur les bras espaceurs organiques avant greffage. On peut aussi réaliser leur fixation, après greffage et dans ce cas, le procédé comprend de plus une étape de fixation des cyclopeptides sur les bras espaceurs organiques, après greffage de ceux-ci.

D'autres caractéristiques et avantages de l'invention apparaîtront mieux à la lecture de la description qui suit, d'exemples de réalisation donnés bien entendu à titre illustratif et non limitatif, en référence aux dessins annexés.

### Brève description des dessins

La figure 1 représente le premier mode de synthèse d'un cyclopeptide conforme à l'invention.

La figure 2 représente le second mode de synthèse d'un cyclopeptide conforme à l'invention.

La figure 3 illustre la liaison d'un cyclopeptide conforme à l'invention sur un support au moyen d'un bras espaceur organique.

La figure 4 illustre la fixation d'un composé organique portant deux cyclopeptides sur un support par l'intermédiaire d'un bras espaceur.

### Exposé détaillé des modes de réalisation

On décrit ci-après la synthèse chimique des peptides P1 à P22 décrits dans le tableau 1, qui comportent des séquences correspondant aux segments β5-β6 du facteur de croissance de l'endothélium vasculaire (VEGF-A).

Les peptides P1 à P6, P10, P14 et P15 sont des peptides linéaires et les peptides P7 à P9, P11, P12, P13 et P16 à P24 sont des cyclopeptides.

Les cyclopeptides P7 à P9, P11, P12, P13, P16 à P18 et P23 sont préparés en utilisant la méthode de synthèse A, soit le premier mode de réalisation du procédé de synthèse des cyclopeptides de l'invention.

Les cyclopeptides P12, P13 et P19 à P22 sont préparés en utilisant la méthode de synthèse B, soit le second mode de réalisation du procédé de synthèse des cyclopeptides de l'invention.

Dans la description qui suit, de ces synthèses, on a utilisé les abbréviations suivantes :
- Fmoc : 9-fluorénylméthoxy carbonyle,
- tBu : t-butoxycarbonyle,
- Trt : trityle,
- 2-Cl Trt : 2-chlorotrityle,
- HMPB : acide 4-hydroxyméthyl-3-méthoxyphénoxybutyrique
- BHA : benzhydrylamine.
- HOBt : N-hydroxybenzotriazole,
- DCC : N,N'-dicyclhexylcarbodiimide,
- DCM : dichlorométhane,
- TFA : acide trifluoroacétique,
- DIEA : N,N-diisopropyl-éthylamine,
- NMM : N-méthylmorpholine,
- PyBoP : Triazole-1-yl-oxy-tris-pyrrolidinophosphonium hexaflorophosphate,
- NMP : N-méthylpyrrolidone,
- DIPCDI : N,N-diisopropylcarboiimide,
- DMAP : 4-diméthylaminopyridine,
- FCS : Sérum foetal de veau,
- PBS : tampon salin au phosphate,
- DMEM : Milieu essentiel minimal modifié selon Delbecco,
- HEPES : acide[N-2-(hydroxyéthyl)pipérazine-N'-(2-éthane sulfonique)].

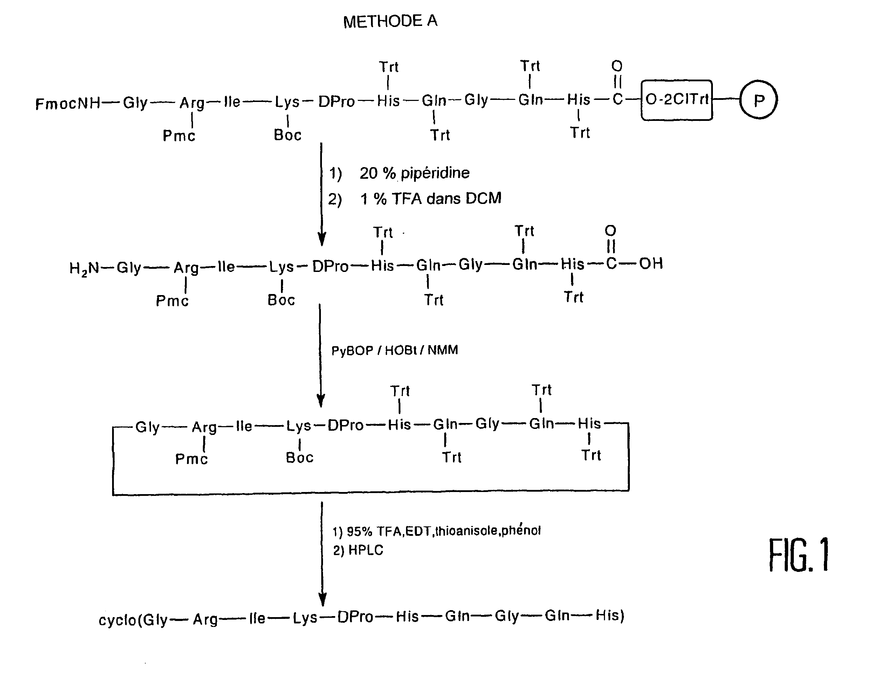

La méthode de synthèse A est représentée sur la figure 1 qui illustre la synthèse du cycopleptide P7.

Selon cette méthode, on synthétise tout d'abord le peptide linéaire sur une phase solide P par synthèse par lots en utilisant la technique de protection Fmoc/tBu, et un synthétiseur automatique Applied Biosystems 430A. Pour la préparation des fragments peptidiques protégés, on utilise des résines préchargées 2-chlorotrityle labile aux acides, par exemple les résines H-His(Trt)-2-ClTrt et H-Ile-2-ClTrt, ou des résines de HMPB-BHA à base de BHA polystyrène fonctionnalisés avec un linker qui est l'acide 4-hydroxyméthyl-3-méthoxy-phénoxybutanoïque de Rinker, par exemple la résine Fmoc-Gly-HMPB-BHA.

Les acides aminés protégés par le groupe 9-fluorénylméthoxycarbonyle (Fmoc) sont couplés en utilisant un excès correspondant à quatre fois la quantité d'aminoacide activé en tant qu'ester de N-hydroxybenzotriazole (HOBt) au moyen de N, N'-dicyclohexylcardodiimide (DCC).

Sur la figure 1, la première ligne représente le peptide linéaire dont les chaînes latérales sont protégées soit par des groupes trityle (Trt) dans le cas des groupes amino de His et Gln, le groupe Boc (t-butyloxycarbonyle) dans le cas du groupe amino de Lys et le groupe 2,2,5,7,8-pentaméthyl-chromane-6-sulfonyle (Pmc) dans le cas de Arg.

On procède alors à la séparation du peptide linéaire de la phase solide P par traitement avec une solution à 1 % d'acide trifluoroacétique (TFA) dans du dichlorométhane (DCM).

On obtient ainsi un fragment peptidique protégé avec un haut rendement et un haut degré de pureté et une perte négligeable des groupes protégeant les chaînes latérales des aminoacides.

Un traitement répété de la résine peptidylique avec une solution fraîche de TFA et des temps de réactions minimaux (dix fois pendant deux minutes) donne les meilleurs résultats. On lave alors la résine avec DCM et du méthanol plusieurs fois. L'achèvement de la coupure est suivi par chromatographie sur couche mince. Les filtrats combinés sont évaporés sous pression réduite et de l'eau glacée est ajoutée au résidu pour précipiter le matériau peptidique. Le matériau brut est isolé par filtration, lavé avec de l'eau fraîche et séché dans un dessiccateur sous vide poussé sur NaOH. Les peptides linéaires protégés sont analysés par chromatographie liquide à haute performance HPLC sur phase inverse (colonne Lichrosob RP-18 de Merck, 7 *µ*m, 0,4 x 25 cm) en utilisant un gradient linéaire allant de 70 à 100 % de B dans A, le solvant A étant une solution aqueuse à 0,1 % de TFA et le solvant B étant une solution aqueuse à 0,1 % de TFA et 70 % d'acétonitrile.

Le peptide linéaire protégé correspondant à P7 est illustré sur la figure 1.

On procède ensuite à la cyclisation du peptide linéaire protégé. Dans ce but, on le dissout dans du DCM pour obtenir une concentration finale de 1 mg/ml. On ajoute à la solution 6 équivalents de N,N-diisopropyléthylamine (DIEA) ou de N-méthylmorpholine (NMM), et on réalise la cyclisation au moyen de benzotriazole-1-yl-oxy-tris-pyrrolidino-phosphonium hexafluorophosphate (PyBOP)/N-hydroxybenzotriazole (HOBt) en utilisant trois équivalents de chaque réactif de couplage. On maintient le milieu réactionnel à 25°C sous atmosphère d'azote et faible agitation pendant 24 à 48 heures jusqu'à ce que la cyclisation soit complète, ce qui est vérifié par HPLC.

On élimine le solvant par évaporation sous pression réduite et on ajoute de l'eau glacée au résidu. On sépare le précipité brut par filtration, on le lave à l'eau fraîche, on le sèche dans un dessicateur sous vide poussé sur NaOH, et on l'utilise pour la déprotection totale des chaînes latérales sans purification complémentaire.

Le cyclopeptide protégé correspondant à P7 est illustré sur la figure 1.

La déprotection des chaînes latérales du cyclopeptide est effectuée au moyen d'une solution de TFA à 95 % en présence des réactifs (phénol, thioanisole, triisopropylsilane, eau) pendant 2 à 3 heures. On effectue ensuite une évaporation de la majeure partie du TFA pour obtenir une bonne précipitation du cyclopeptide brut déprotégé, et on ajoute 10 fois le volume d'éther refroidi à la glace. Après filtration et lavage avec de l'éther frais, on sèche le précipité sur NaOH et on lyophilise. On effectue ensuite des purifications semi-préparatives sur une colonne HPLC d'Applied Biosystems en utilisant la colonne à phase inverse Lichrosorb C18 (250 x 10). On utilise les tampons A et B décrits ci-dessus. Des gradients linéaires de 10 à 50 % de B dans A sur une durée de 30 minutes, à un débit de 4 ml/min, sont utilisés. On réunit les fractions homogènes obtenues par HPLC et on les lyophilise pour obtenir les cyclopeptides voulus avec un taux de pureté satisfaisant, supérieur à 95 % par HPLC analytique.

La figure 1 illustre le cyclopeptide protégé, puis l'étape de déprotection aboutissant au cyclopeptide P7 du tableau 1.

On suit le même mode opératoire pour préparer les peptides P8, P9, P11, P12, P13, P16 à P18 et P23, en utilisant pour la synthèse les acides aminés L ou D protégés par le groupe Fmoc, la résine Fmoc-L-Gly-HMPB/BHA (0,53 mmol/g), la résine H-His(Trt)-2-ClTrt (0,42 mmol/g), la résine H-Ile-2-ClTrt (0,53 mmol/g), et la résine NovaSyn TGA ((90 *µ*m), et PyBOP provenant de la Société Novabiochem.

On utilise aussi les réactifs HOBt, DCC, TFA, DIEA, NMM et la pipéridine provenant de Aldrich.

On précise que les HPLC sont effectuées sur colonne Lichrosorb RP-18 (7-µm, 0,4 x 25 cm) de Merck, et sur colonne Lichrosorb C-18 (5-*µ*m, 0,4 x 25 cm) de Interchim, en réalisant l'élution avec un gradient linéaire du solvant B dans le solvant A. Le solvant A comprend 0,1 % de TFA dans H₂O. Le solvant B comprend 0,1 % de TFA, 70 % d'acétonitrile et 30 % d'eau. On utilise un débit de 1 ml/min sur une durée de 30 minutes. La détection UV est effectuée à 214 nanomètres et 280 nanomètres. Les peptides purifiés sont caractérisés par analyse des aminoacides, en réalisant l'hydrolyse dans HCl 6M et du phénol à 2 %, à 110°C, pendant 20 à 24 heures, et par spectrométrie de masse FAB-MS.

On décrit maintenant la méthode de synthèse B, soit le second mode de réalisation du procédé de synthèse des cyclopeptides de l'invention.

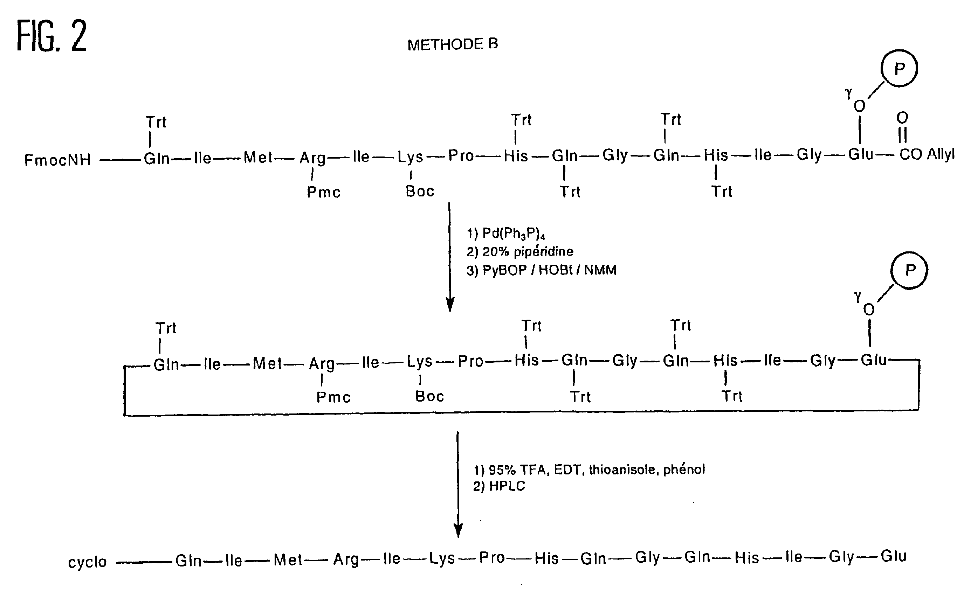

Le mode de synthèse B est illustré sur la figure 2 dans le cas du peptide P19.

Pour cette synthèse, on utilise les mêmes réactifs que pour la synthèse A en y ajoutant la résine Fmoc-L-Glu (PEG-PS)-OAllyl (0,18 mmol/g) de Perkin Elmer.

On synthétise tout d'abord le peptide linéaire P10 par synthèse en phase solide en utilisant le synthétiseur automatique Applied Biosystems 430 A, comme dans la méthode de synthèse A.

Comme représenté sur la figure 2, le premier acide aminé C-terminal Fmoc-Glu-OAllyl est fixé sur la résine NovaSyn TGA de 90 *µ*m (phase solide P) par sa chaîne latérale en utilisant une méthode à anhydride symétrique. On fait gonfler la résine dans de la N-méthylpyrrolidone (NMP) pendant 30 minutes. On ajoute une solution de 5 équivalents de N,N-diisopropylcarbodiimide (DIPCDI), à une solution de 10 équivalents de l'acide aminé dans du DCM sec à 0°C. Après agitation pendant 30 minutes, on élimine le solvant sous pression réduite et on dissout le résidu dans NMP et on le transfère sur la résine pré-imprégnée. La fixation de l'acide carboxylique est achevée par estérification catalysée par la 4-diméthylaminopyridine (DMAP), à raison de 0,1 équivalent pendant 2 heures, pour donner une substitution finale de 0,25 mmol/g. La charge de la résine est estimée par quantification UV du produit d'addition pipéridine-Fmoc à 290 nm. La résine commerciale Fmoc-L-Glu-(PEG-PS)-OAllyl (0,18 mmol/g) provenant de Perkin Elmer a aussi été utilisée.

On couple les acides aminés suivants, protégés par le groupe Fmoc, en utilisant un excès correspondant à 4 fois la quantité d'aminoacide activé en tant qu'ester HOBt au moyen de DCC.

On obtient ainsi le peptide linéaire à chaînes latérales protégées, représenté à la première ligne de la figure 2.

On effectue ensuite une déprotection allylique de l'acide aminé terminal Glu fixé sur la phase solide, en traitant la résine avec le peptide protégé par 3 équivalents de [Pd(PPh₃)₄]. dans un mélange DCM : AcOH : NMM (37 : 2 : 1) sous un courant d'argon à 25°C pendant 2,5 heures sous agitation douce occasionnelle. On élimine le catalyseur en lavant la résine avec 0,5 % de DIEA dans NMP, pendant 2 minutes 3 fois dans NMP, puis dans du diéthyldithiocarbamate de sodium à 0,5 % P/P dans NMP, 3 fois pendant 15 minutes, et finalement dans NMP et DCM pendant 2 minutes en répétant 3 fois.

On effectue ensuite la cyclisation du peptide sur la phase solide de la façon suivante. On traite la résine avec 20 % de pipéridine dans NMP pour libérer le groupe amino N-terminal du peptide et on la lave avec HOBt 1N dans NMP, puis avec NMP et DCM. On effectue la cyclisation en phase solide en utilisant 6 équivalents de PyBOP, 6 équivalents de HOBt et 12 équivalents de DIEA dans NMP, pendant 4 à 6 heures. On vérifie l'achèvement de la cyclisation par le test à la ninhydrine. On lave la résine plusieurs fois avec MMP et DCM, et on la sèche sous vide poussé pendant une nuit. Le cyclopeptide protégé est représenté sur la figure 2.

On procède ensuite à la séparation du cyclopeptide de la phase solide et à la déprotection des chaînes latérales des aminoacides. Ceci est effectué au moyen de TFA à 95 % et le produit d'acidolyse est purifié par HPLC semi-préparative comme décrit ci-dessus.

On réunit les fractions homogènes obtenues par HPLC et on les lyophilise pour obtenir les cyclopeptides voulus avec un degré de pureté satisfaisant (supérieure à 95 % par HPLC analytique).

On obtient ainsi le cyclopeptide P 19 représenté sur la figure 2.

On suit le même mode opératoire pour préparer les cyclopeptide P20 à P22 du tableau 1.

Les peptides linéaires P1 à P6, P10, P14 et P15 du tableau 1 sont préparés par synthèse peptidique classique.

Le tableau 2 illustre la structure et les masse moléculaire des peptides P1 à P22 en se référant à la séquence peptidique du VEGF-A qui comporte 165 acides aminés.

On teste les peptides P1 à P24, en ce qui concerne leur propriété d'inhibition de la liaison du VEGF sur son récepteur KDR.

Pour ces essais, on utilise des cellules d'ovaires d'hamster chinois déficients en sulfate d'héparane transfectées avec un vecteur d'expression contenant VEGFR2cDNAs (Cellules CHOm VEGFR2). Il a été démontré que le KDR recombinant des cellules CHO présente les mêmes caractéristiques que le KDR de cellules endothéliales (voir Binétruy-Tournaire et al, EMBO Journal, 19, (7), 2000 pages 1525-1533 [10]).

Les cellules CHOm VEGFR2 sont obtenues par transfection des cellules d'ovaires d'hamsters chinois déficients en sulfate d'héparane avec du VEGFR2 cDNA dans un vecteur psV-7d comme il a été décrit par Jonca et al dans J. Biol. Chem., 1997, 272, pages 24203 [11]. Les cellules CHOm VEGFR2 sont cultivées en routine dans un milieu Eagle modifié par un milieu Dulbecco, supplémenté avec 10 % (V/V) de sérum foetal de veau (FCS), 50 U/ml de pénicilline, 50 *µ*g/ml de streptomycine, 1 mg/ml de glucose et 2 mM de L-glutamine, à 37°C, dans une atmosphère à 5 % de CO₂.

Pour ces essais, on utilise du VEGF marqué radioactivement avec ¹²⁵I-Na en utilisant des perles d'iode (Iodogen).

L'activité spécifique de ¹²⁵I-VEGF est de 150000 à 200000 cpm/ng. Les cellules sont ensemencées dans des boîtes de 3,5 cm de diamètre, revêtues au préalable de 0,15 % de gélatine, à une densité de 200000 cellules par boîte et cultivées dans un milieu complet pendant 2 jours. Les boîtes subconfluentes sont transférées à 4°C. Les cellules sont lavées deux fois avec un tampon salin au phosphate refroidi à la glace (PBS) et incubées avec différentes concentrations de peptides et 5 ng/mL de ¹²⁵I-VEGF, en présence ou en l'absence de 50 ng/mL d'héparine, dans un tampon de liaison (DMEM contenant 20 mmol/L de HEPES, pH 7,4, 0,15 % de gélatine), pendant 2 heures, à 4°C. A la fin de la période d'incubation, les cellules sont lavées 3 fois avec du PBS refroidi à la glace et solubilisées dans 1 mL de tampon avec 2 % de Triton X 100, 10 % de glycérol, 1 mg/mL de sérum albumine-bovine BSA. Le taux de radioactivité liée aux cellules est compté dans un compteur gamma Kontron MR-250. La liaison non-spécifique est déterminée par incubation des cellules avec ¹²⁵I-VEGF et un excès de 200 fois de VEGF non marqué. La liaison spécifique est calculée en soustrayant la liaison non spécifique de la liaison totale.

On effectue cet essai, en présence de concentrations fixées des peptides P1 à P22, de 20 *µ*M, 200 *µ*M, et 400 *µ*M. Tous les peptides linéaires et quelques peptides cycliques ne montrent pas d'effet inhibiteur sur la liaison du VEGF au KDR à ces concentrations. On poursuit les essais avec les cyclopeptides intéressants et deux peptides linéaires comme témoins pour une analyse de l'inhibition en fonction de la dose.

Les résultats obtenus représentés par IC₅₀, soit la concentration en µM nécessaire pour inhiber 50 % de la liaison du VEGF au récepteur KDR, sont donnés également dans le tableau 2.

Tous les essais sont répétés trois fois et donnent des résultats similaires.

On remarque ainsi que les cyclopeptides P11, P16, P17, P19, P20, P23 et P24 sont très efficaces pour inhiber la liaison de VEGF au récepteur KDR.

En revanche, le cyclopeptide P22 qui correspond au cyclopeptide décrit dans US-A-5 939 383 [8] n'est pas plus efficace que les peptides linéaires P1 à P6, P10, P14 et P15 pour inhiber cette liaison.

Les peptides inhibant la liaison du ¹²⁵I-VEGF au récepteur VEGFR2 sont ensuite testés dans des expériences d'angiogenèse in *vitro, ex vivo* et in vivo et sur leur effet sur la signalisation cellulaire. On donne ici des résultats concernant l'effet des peptides actifs sur la prolifération cellulaire, la migration cellulaire et sur l'activation des kinases p42 et p44 (MAP kinases : kinases activées par les mitogènes).

La prolifération cellulaire est mesurée par comptage cellulaire. 7000 cellules endothéliales sont placées dans des puits de plaques de 24 (Costar) dans du milieu DMEM contenant du sérum bovin de nouveau né. Après que les cellules ont adhéré, les cellules sont lavées dans du DMEM sans sérum et incubées avec du milieu DMEM contenant 1 % de sérum bovin de nouveau-né, 10 ng/mL de VEGF en présence ou en absence de différentes concentrations de peptides. Deux jours après, les cellules sont encore une fois stimulées par le VEGF en présence ou absence de différentes concentrations de peptide. Au cinquième jour, les cellules sont trypsinisées et comptées avec un compteur Coulter. A titre d'exemple, le peptide P11 (circulaire) montre une forte inhibition de la prolifération cellulaire. En revanche, le peptide linéaire est inactif (Tableau 3).

La migration cellulaire est mesurée selon la méthode décrite par Sato et Rifkin (J. Cell Biol., Sept., 1988, (107 (3) : 1199-205) [17] avec des modifications. 100 000 cellules endothéliales sont placées dans des boites de culture de 35 mm dans du milieu DMEM contenant 10 % de sérum de veau foetal. A confluence, le milieu est remplacé par du milieu DMEM sans sérum et les cellules sont laissées incuber la nuit. Le lendemain, une dénudation est artificiellement effectuée dans la monocouche avec une pointe de pipette stérile. Une série de photos digitales est prise et le bord de la dénudation est tracé par une ligne utilisant le programme BiocomVisionL@b2000. Les boîtes sont ensuite rincées et incubées avec 10 ng/mL de VEGF en présence ou absence de peptide. Après 18 heures d'incubation, une nouvelle série de photos est prise et les images avant et après stimulation superposées. Les cellules se trouvant au-delà du bord de dénudation sont comptées. Par exemple, Le peptide P11 (cyclique) dans ce type d'expérience inhibe fortement la migration cellulaire. En revanche, le peptide linéaire n'a aucun effet (Tableau 3).

La phosphorylation de ERK1 (p44) et de ERK2 (p42) est mesuré par Western Blot utilisant des anticorps anti p42/p44 (New England Biolabs). Les cellules endothéliales capillaires sont cultivées en DMEM contenant 10 % de sérum bovin de nouveau-né . Des cultures subconfluentes sont ensuite dépourvues de sérum pendant 24 heures. Les peptides sont rajoutés à des concentrations spécifiques pendant 5 minutes aux cellules en présence ou en absence de 10 ng/mL de VEGF. Les cellules sont' ensuite enlevées des boîtes de culture et lysées pendant 20 minutes sur la glace dans du tampon de lyse contenant 50 mM Hepes, pH 7,4, 75 mM NaCl, 1 mM EDTA, 1% Nonidet P-40 et 0,01 % de SDS. Le matériel insoluble est enlevé par centrifugation et 50 µg/mL d'extrait protéique sont séparées par l'électrophorèse en gel de polyacrylamide contenant du sodium dodécyl sulfate (SDS) dans des conditions dénaturantes. Les protéines sont ensuite transférées du gel sur une membrane de nitrocellulose (Amersham Pharmacia Biotech, Orsay) avec un appareil de transfert (Transfert semi-sec, Bio-Rad, lvry-sur-Seine, France). La membrane est ensuite incubée avec les anticorps primaires contre p42/p44 puis secondaires couplés à la peroxydase. La visualisation est effectuée en utilisant le système ECLplus (Amersham Pharmacia Biotech, Orsay). Les résultats sont quantifiés en utilisant le programme Image Quant (Molecular Dynamics).

A titre d'exemple, le peptide P11 (cyclique) inhibe fortement la phosphorylation de p42/p44. Le peptide linéaire n'a pas d'effet (Tableau 3).

**Tableau 3**

| | **Prolifération (IC50)** | **Migration (IC50)** | **Phosphorylation p42 (% d'inhibition)** | **Phosphorylation p44 (% d'inhibition)** |
|---|---|---|---|---|
| P11 | 7 µM | 30 µM | 75 % | 90 % |
| P14 | >300 µM | >300 µM | 0% | 0% |

Le tableau 3 indique l'effet du peptide P11 sur la prolifération et la migration cellulaire et sur la phosphorylation des kinases MAP (p42 et p44). Les expériences ont été effectuées comme indiquées. L'inhibition de la prolifération et de la migration est indiquée en valeurs d'inhibition à 50 % de l'effet biologique (IC 50). 50 µM de peptides ont été utilisés pour les expériences de phosphorylation. Le degré de phosphorylation est estimé après quantification des signaux obtenus à partir des films d'autoradiographie et les résultats sont exprimés en pourcentage d'inhibition par rapport au signal obtenu avec 10 ng/mL de VEGF seul.

Les cyclopeptides de l'invention peuvent être utilisés pour la réalisation de systèmes inhibiteurs ou activateurs de l'angiogenèse, soit sous forme soluble, soit en les couplant à des supports appropriés.

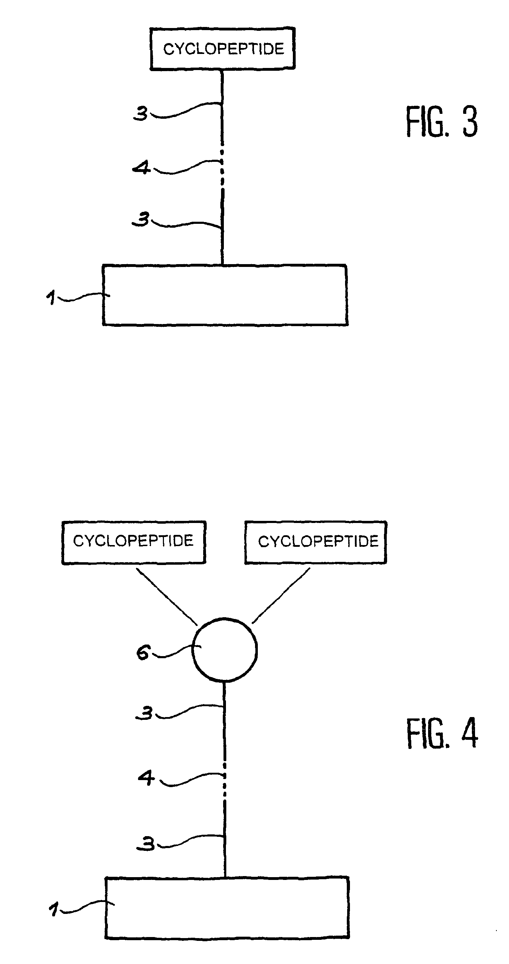

Sur la figure 3, on a représenté le premier mode de réalisation d'un système inhibiteur conforme à l'invention.

Sur cette figure, on voit que le cyclopeptide est couplé à un support approprié 1 par l'intermédiaire d'un bras espaceur organique 3, qui peut comporter un motif 4 susceptible d'être coupé par un système enzymatique.

Le bras espaceur organique peut être constitué par une chaîne hydrocarbonée, fluorocarbonée, polyéther, polyéthylène glycol, polyamine, polyamide, polyester, polysiloxane ou une combinaison de celles-ci, qui est fonctionnalisée à chaque extrémité pour former une liaison covalente, d'une part, avec le cyclopeptide et, d'autre part, avec le support 1.

Le motif 4 susceptible d'être coupé par un système enzymatique présent dans le bras espaceur 3 peut être en particulier une séquence peptidique qui est un substrat des enzymes telles que les métalloprotéases de la matrice extra-cellulaire.

La présence de ce motif permet ainsi de libérer le cyclopeptide à l'endroit voulu lorsqu'il est en contact avec les enzymes appropriées, permettant ainsi au cyclopeptide de remplir sa fonction d' inhibition de la liaison du VEGF au récepteur KDR et de maîtriser l'angiogenèse.

Sur la figure 3, on a représenté un seul cyclopeptide lié au support. Bien entendu, on peut utiliser des supports comportant un grand nombre de cyclopeptides, liés chacun au support par un bras espaceur organique comportant ou non un motif tel que 4.

Sur la figure 4, on a représenté un autre mode de réalisation des systèmes de l'invention, dans lequel on utilise deux cyclopeptides pour obtenir un effet activateur des récepteurs KDR.

Dans ce cas, deux cyclopeptides sont fixés sur un composé 6 qui est lui-même relié au support 1 par un bras organique espaceur 3 ; ce bras peut également comporter un motif 4 susceptible d'être coupé par un système enzymatique.

Le choix du composé organique sur lequel sont fixés les deux cyclopeptides permet de disposer ceux-ci dans une configuration satisfaisante pour autoriser la dimérisation des récepteurs du facteur de croissance de l'endothélium vasculaire.

Ainsi, la distance entre les deux cyclopeptides fixés est telle que lorsque le système est mis en contact avec des cellules exprimant des récepteurs du VEGF, il autorise la dimérisation de ces récepteurs.

Le bras organique espaceur 3 peut être du même type que celui de la figure 3 et comporter un motif 4 du même type que celui de la figure 3.

Le composé organique 6 peut être formé de séquences alternées hydrophiles-hydrophobes, du type polyéthylèneglycol/alcane ou fluoroalcane, pour permettre une disposition appropriée des deux cyclopeptides. La fixation de chaque cyclopeptide sur ce composé met en oeuvre des fonctions amines ou acides carboxyliques des chaînes latérales du cyclopeptide ou des fonctions de type acrylique.

Le support 1 utilisé dans les différents modes de réalisation des systèmes de l'invention peut être sous forme solide ou sous forme de gel. Il peut s'agir d'un solide organique ou inorganique.

De préférence, le support est un polymère organique biocompatible, biodégradable ou non. Dans ce cas, la fixation du bras espaceur organique sur ce polymère peut être effectuée par voie chimique ou radiochimique. Dans ce dernier cas, on soumet le support en polymère organique à une irradiation au moyen de rayonnements ionisants, de plasmas ou de photons sur les zones définies du support qui devront recevoir le bras espaceur, et on greffe ensuite sur ces zones le bras espaceur organique, soit directement, soit par l'intermédiaire d'un monomère polymérisable par voie radicalaire (radiogreffage) comportant par exemple des fonctions amine ou acide carboxylique.

Le couplage au polymère radiogreffé ou non se fait par exemple par création de liaisons amides, soit entre les fonctions acides carboxyliques et les fonctions amines introduites à l'extrémité des bras espaceurs, soit entre les fonctions amines du support et les fonctions acides carboxyliques introduites à l'extrémité des bras espaceurs.

Les exemples suivants illustrent la réalisation de tels systèmes.

### Exemple 1 : Réalisation d'un système comportant deux cyclopeptides reliés de manière covalente par un composé organique.

On part d'un composé organique comportant trois groupes fonctionnels dont l'un est protégé.

Le composé organique répond à la formule :

HOOC CH₂CH₂O(CH₂CH₂O)₅(CH₂)₃NH(CH₂CH₂O)₅CH₂CH₂ COOH (composé 1)

Le bras espaceur est fixé sur le groupe NH de ce composé, et il répond à la formule :

CH₂ = CH-CO-NH-(CH₂)₅-CO-P-CO- (composé 2)

dans laquelle P représente le peptide qui est un substrat de métalloprotéase de la matrice extracellulaire.

On prépare le composé organique 1 en effectuant les étapes suivantes.

### Etape n°1 : Condensation de l'hexaéthylèneglycol sur l'acrylate de tertiobutyle.

Pour cette étape, on suit le mode opératoire décrit par O.Seitz, H. Kunz, J. Org. Chem., 62, 813 (1997) [12] :

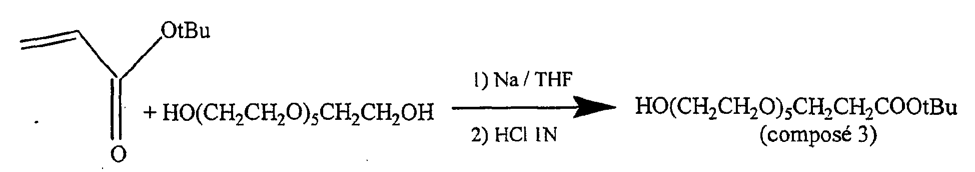

Dans un tricol, on ajoute le THF anhydre avec l'hexaéthylène glycol le tout sous azote et agitation. Qn ajoute alors Na que l'on laisse dissoudre. On rajoute l'acrylate de tertiobutyle. On laisse tourner 20 heures à température ambiante. On ajoute HCl 1N pour neutraliser la solution. On évapore le THF sous pression réduite, puis on noie la solution dans de l'eau salée saturée. La solution est alors extraite à l'acétate d'éthyle trois fois. L'ensemble des phases organiques est de nouveau noyé dans de l'eau salée saturée. On récupère la phase organique et on évapore l'acétate d'éthyle. On récupère le produit pur (composé 3) avec un rendement de 96 %.

### Etape n°2 Fonctionnalisation de l'autre extrémité du composé 3 par azidation.

- a) tosylation selon le mode opératoire décrit par D.S. Wlibur et al, Bioconjugate Chem., 9, 813 (1998) [13] : 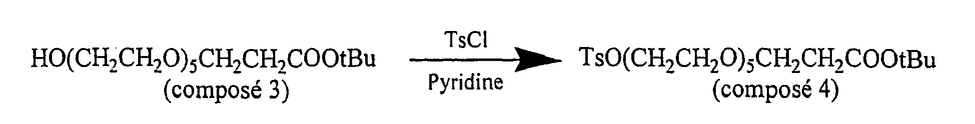
   Dans un dicol, on place la pyridine et le composé 3 de départ. Le tout est placé à 0°C sous N₂. On rajoute alors le TsCl. Après 15 heures de réaction, la solution est noyée dans la glace et extraite 3 fois au CH₂Cl₂. La phase organique est lavée avec une solution d'acide acétique 2 %, puis avec de l'eau. La phase organique est alors récupérée, séchée sur MgSO₄ et évaporée sous pression réduite. Le produit est purifié par passage sur colonne de silice (70/230) avec 100 % d'acétate d'éthyle comme éluant. Le produit (composé 4) est récupéré avec un rendement de 65 %.
- b) Azidation selon le mode opératoire décrit par K. D. Reynolds, Bioconjugate Chem., 10, 1021-31 (1999) [14] :

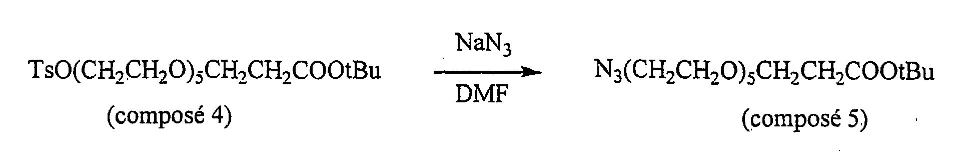

Dans un dicol, on place le composé 4 avec du DMF sous N₂, puis on ajoute NaN₃. On laisse tourner le tout pendant 20 heures, la solution devient opaque. La solution est passée sur fritté, puis le DMF est coévaporé avec du toluène. On obtient un précipité blanc que l'on dilue à l'éther. La solution est de nouveau passée sur fritté et l'éther est évaporé.

On obtient alors le composé 5 avec un rendement de 95 %.

### Etape n°3 : Fontionnalisation de l'autre extrémité du composé 3 par allylation.

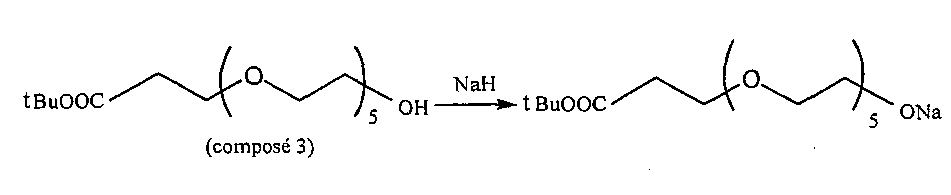

L'allylation est réalisée par substitution nucléophile du bromure d'allyle commercial par le sel de sodium de l'hexaéthylèneglycol. Le dérivé obtenu est 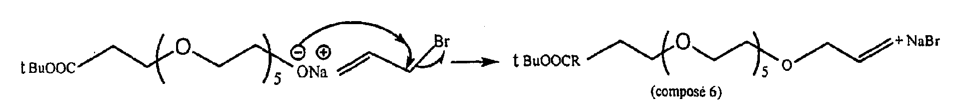 ensuite isolé par chromatographie sur colonne de silice avec un rendement de 50 %.

La synthèse est effectuée dans le THF.

### Etape n°4 : Hydroboration

Elle est effectuée, selon le procédé décrit par Carboni et al, J. Org. Chem., 1993, 58, 3736-3741 [15] en faisant réagir le composé 5 avec le composé 6 sur lequel a été additionné du dichloroborane. On obtient le composé 7.

tBuOOCCH₂CH₂O(CH₂CH₂O)₅CH₂CH₂CH₂HN(CH₂CH₂O)₅CH₂CH₂-COOtBu (Composé 7)

### Etape n°5 Protection de l'azote

Le compose 7 est traité par du FmocOSu, selon le procédé utilisé par exemple par Chetyrkina et al., Tetrahedron Letters 2000, 41, 1923-1926 [16].

On obtient le composé 8

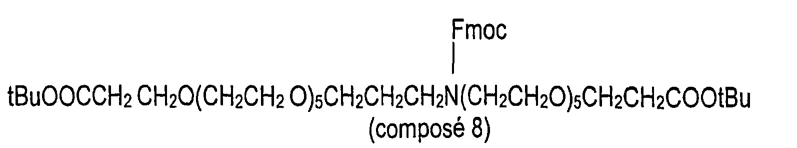

### Etape n°6 : Déprotection des groupes tertiobutyloxycarbonyle.

Dans un dicol, on place le composé 8 auquel on ajoute l'anisole, puis le TFA. On laisse tourner la réaction pendent 1h30 minutes à 25°C, puis on évapore le TFA sous pression réduite. Le brut est alors passé sur colonne de silice (70/230) avec comme élulant 95/5. CH₂Cl₂/MeOH, puis 90/10 CH₂Cl_{2/}MeOH. On récupére le composé 9 pur avec un rendement de 65 % .

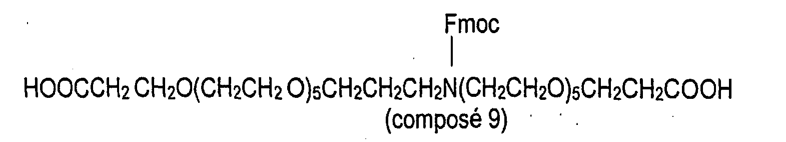

On fixe les cyclopeptides P23 sur les deux groupes fonctionnels (COOH) du composé 9 en opérant de la manière suivante.

On place le composé 9 en solution dans du THF et on l'active au moyen de N,N-diméthylpropyl,éthylcarbodiimide.

On ajoute alors une quantité correspondant à 1 équivalent molaire de chaque cyclopeptide et on laisse le milieu pendant 12 heures à la température ambiante, puis on lyophilise. On reprend le lyophilisat par du chloroforme, on élimine l'urée qui s'est formée par filtration, et on évapore le filtrat sous pression réduite.

On caractérise le produit obtenu par spectrométrie infrarouge à transformée de Fourier, RMN du proton et spectrométrie de masse. Il correspond au produit recherché.

Pour assurer la fixation de ce produit sur un support, on fixe tout d'abord sur le groupe NH du composé organique un bras espaceur comportant une fonction polymérisable. On procède de la manière suivante.

### Déprotection du groupe NH

On réalise tout d'abord la déprotection du groupe NH du composé 9 par traitement par la pipéridine.

On prépare par ailleurs le bras espaceur en effectuant la réaction suivante :

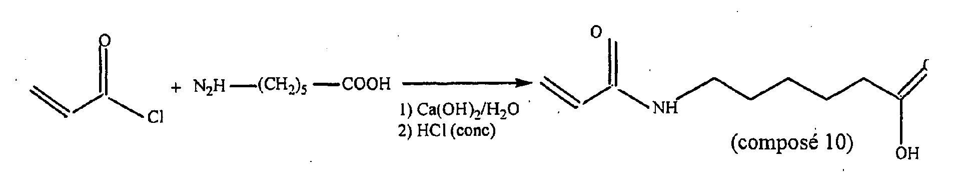

Dans un tricol on ajoute Ïe sel et l'eau, puis l'aminoacide H₂N (CH₂)₅-COOH ; le tout est refroidi à 0°C. Sous forte agitation, on ajoute le chlorure de l'acide acrylique, à raison d'environ 3 mL par minute.

On laisse tourner 10 minutes à température ambiante, puis on passe la solution sur fritté. Le ballon est remis à 0°C et sous forte agitation. On ajoute alors HCl concentré pour obtenir un pH = 2. Ceci provoque une précipitation du produit. Le mélange est alors filtré et le produit séché (composé 10) est pur. Le rendement est de 75 %

### Condensation du composé 10.

On condense le composé 10 sur le peptide P qui est un substrat de métalloprotéase de la matrice extracellulaire, lors de la dernière étape de sa synthèse en phase solide, puis l'ensemble est libéré de la résine sans déprotection des chaînes latérales.

La molécule obtenue est alors condensée grace au dicyclohexyl carbodiimide sur le composé 9 déprotégé. On déprotège alors les chaînes latérales du peptide du bras espaceur, puis on fixe le bras sur un support en polymère par l'intermédiaire des fonctions acryliques du bras.

On utilise comme support un film industriel de 25 µm d'épaisseur en polytéréphtalate d'éthylène (PTE) et on l'extrait préalablement au Soxhlet par du toluène à reflux, à une température de 110°C, pendant 12 heures, afin d'éliminer toute trace de monomère organique.

On place ensuite sur le film de polymère une grille métallique en nickel ou en cuivre, d'une épaisseur de 50 *µ*m, percée de trous circulaires d'un diamètre de 5 à 10 *µ*m répartis régulièrement sur toute sa surface, avec un pas d'environ 100 *µ*m. La grille a été fabriquée par électroformage pour présenter une reproductibilité parfaite et une très haute précision de l'ordre de micromètre.

On soumet ensuite le film de polymère à une irradiation au moyen d'un faisceau d'électrons pour irradier uniquement les zones correspondant aux trous de la grille. Le faisceau d'électrons présente les caractéristiques suivantes :
- énergie Ep = 2,5 MeV,
- intensité maximale Imax = 1 *µ*A,
- dose maximale = 500 kGy.

On effectue l'irradiation sous atmosphère contenant de l'oxygène.

Après irradiation, on retire la grille du film de polymère et on met le film de polymère irradié en contact avec le composé organique sur lequel sont fixés les deux cyclopeptides pour greffer ce composé, par l'intermédiaire du bras espaceur, sur le film de polymère. Cette fixation est effectuée en mettant en contact le film avec une solution 10⁻² M du composé organique obtenu précédemment, dans de l'acétonitrile, en opérant à 40°C.

On greffe ainsi le composé organique par les fonctions acryliques du bras espaceur sur les zones irradiées du film de polymère.

On caractérise le produit final par spectrométrie FT-IR et XPS.

### Exemple 2 : Préparation d'un système comportant deux cyclopeptides.

Dans cet exemple, on suit le même mode opératoire que dans l'exemple précédent pour coupler deux cyclopeptides P23 sur deux fonctions du composé organique décrit, utilisé précédemment.

Pour réaliser le couplage, on place le composé organique en solution dans du chloroforme et on l'active par du dicyclohexylcarbodiimide DCCI, puis on ajoute une quantité correspondant à 1 équivalent molaire de chaque cyclopeptide et on maintient le milieu réactionnel pendant 12 heures à 4°C. On élimine le précipité de dicyclohexylurée par filtration, puis on évapore le filtrat sous pression réduite.

On caractérise le produit obtenu par spectrométrie infrarouge à transformée de Fourier, RMN du proton et spectrométrie de masse, comme précédemment.

On fixe ensuite sur le groupe NH du composé organique un bras espaceur comme dans l'exemple 1.

Puis on fixe l'ensemble sur un support constitué par un film industriel de 25 *µ*m d'épaisseur en poly(fluorure de vinylidène/hexafluoropropylène) PVDF-HFP. On soumet préalablement le film à une extraction au Soxhlet dans du dichlorométhane à reflux, à une température de 40°C, pendant 12 heures, afin d'éliminer toute trace de monomère organique.

On soumet ensuite le film à une irradiation sous air par des ions lourds rapides. Les ions utilisés sont des ions oxygène ayant une énergie primaire Ep d'environ 10 MeV/uma, à des fluences comprises entre 10⁷ et 10⁹ ions/cm² et à des intensités de l'ordre de 10² à 5.10² nA. On choisit un régime à faible fluence de façon à ce qu'il n'y ait pas de recouvrement des traces latentes.

Ce régime est directement déterminé par les paramètres de l'irradiation (numéro atomique de l'ion, énergie primaire, fluence). Dans ce cas, la distribution aléatoire des centres actifs dans la zone perturbée créée lors de l'irradiation (émergence des traces latentes) permet de radiogreffer au moins deux bras espaceurs organiques portant chacun un cyclopeptide, en bloquant la distance entre les points d'ancrage de telle sorte que la distance d entre deux cyclopeptides favorise la dimérisation des récepteurs du VEGF.

Les zones irradiées sont utilisées pour greffer, grâce aux radicaux libres créés le long de la trace latente de l'ion et à l'émergence de celle-ci à la surface du film de polymère, des bras espaceurs organiques portant les cyclopeptides obtenus comme il est décrit ci-dessus, par réaction de leur fonction de type acrylique avec le support irradié.

Ce couplage est effectué par chauffage à 40°C du film irradié mis en présence d'une solution 10⁻³ M des bras organiques porteurs des cyclopeptides, dans le tétrahydrofurane.

On caractérise le composé final par spectrométrie FT-IR et XPS.

### Références citées

[1] : Ferrara et al, Nature Medicine, vol. 5, n°12, Décembre 1999, pages 1361-1364.
[2] : Hagedorn et Bikfalvi, Critical Reviews in Oncology/Hematology, 34, 2000, pages 89-110.
[3] : Muller Y. A., Structure, 1997, 5, n°10, pages 1325-1338.
[4] : Presta et al, Cancer Research, 57 1997, pages 4593-4599.
[5] : Ortega et al, Am. J. Pathol., Nov 1997, 151 (5), pages 1215-1224.
[6] : Piossek et al, The Journal of Biological Chemistry, vol. 274, n°9, 1999, pages 5612-5619.
[7] : Fairbrother et al, Biochemistry 37, 1998, pages 17754-17764.
[8] : US-A-5 939 383
[9] : Bekele et al, J. Org. Chem. 62, 1997, pages 2259-2262.
[10] : Binétruy-Tournaire et al, EMBO Journal, 19, (7), 2000, pages 1525-1533.
[11] : Jonca et al, J. Biol. Chem., 1997, 272, pages 24203.
[12] : O.Seitz, H. Kunz, J. Org. Chem., 62, 813 (1997).
[13] : Wilbur et al, Bioconjugate Chem., 9, 813 (1998).
[14] : J. K. D. Reynolds, Bioconjugate Chem., 10, 1021-31 (1999).
[15] : Carboni et al, J. Org. Chem., 1993, 58, pages 3736-3741.
[16] : Chetyrkina et al, Tetrahedron Letters, 2000, 41, pages 1 923 - 1 926.
[17] : Sato et Rifkin (J. Cell Biol., Sept., 1988, (107 (3) : 1199-205).

**Tableau 1**

| | | |
|---|---|---|
| SEQ ID NO:14 | P1 | H-Arg-Ile-Lys-Pro-His-OH |
| SEQ ID NO:15 | P2 | H-Gln-Ile-Met-Arg-Ile-Lys-Pro-His-OH |
| SEQ ID NO:16 | P3 | H-Arg-Ile-Lys-Pro-His-Gln-Gly-OH |
| SEQ ID NO: 17 | P4 | H-Gln-Ile-Met-Arg-Ile-Lys-Pro-His-Gln-Gly-Gln-His-Ile-Gly-Glu-OH |
| SEQ ID NO:18 | P5 | H-Gln-Ile-Met-Arg-Ile-Lys-Pro-His-Gln-Gly-Gln-His-Ile-Gly-OH |
| SEQ ID NO: 19 | P6 | H-Gly-Arg-Ile-Lys-DPro-His-Gln-Gly-Gln-His-OH |
| SEQ ID NO : 2 | P7 | cyclo(Gly-Arg-Ile-Lys-DPro-His-Gln-Gly-Gln-His) |
| SEQ ID NO: 3 | P8 | cyclo(Gly-Arg-Ile-Lys-Pro-His-Gln-Gly-His) |
| SEQ ID NO:4 | P9 | cyclo(Pro-Arg-Ile-Lys-Pro-His-Gln-Gly-Gln-His) |
| SEQ ID NO:20 | P10 | H-Gln-lle-Met-Arg-lle-Lys-Pro-His-Gln-Gly-Gln-His-lle-Gly-Glu-Oallyle |
| SEQ ID NO : 5 | P11 | cyclo(DPhe-Pro-Gln-Ile-Met-Arg-Ile-Lys-Pro-His-Gln-Gly-Gln-His-Ile-Gly-Glu) |
| SEQ ID NO : 6 | P12 | cyclo(Gly-Gln-Ile-Met-Arg-lle-Lys-Pro-His-Gln-Gly-Gln-His-lle-Gly-Glu) |
| SEQ ID NO : 7 | P13 | cyclo(Pro-Gln-lle-Met-Arg-lle-Lys-Pro-His-Gln-Gly-Gln-His-lle-Gly-Glu) |
| SEQ ID NO:21 | P14 | H- Pro-Gln-Ile-Met-Arg-ile-Lys-Pro-His-Gln-Gly-Gln-His-Ile-Gly-Glu-OH |
| SEQ ID NO:22 | P15 | H-Gly-Gln-Ile-Met-Arg-Ile-Lys-Pro-His-Gln-Gly-Gln-His-Ile-Gly-Glu-OH |
| SEQ ID NO:8 | P16 | cyclo(Arg-Ile-Lys-Pro-His-Gln-Gly) |
| SEQ ID NO ; 9 | P17 | cyclo(Pro-Arg-Ile-Lys-Pro-His-Gln-Gly) |
| SEQ ID NO:23 | P18 | cyclo(DPhe-Arg-lle-Lys-Pro-His-Gln) |
| SEQ ID NO:10 | P19 | cyclo(Gln-lle-Met-Arg-lle-Lys-Pro-His-Gln-Gly-Gln-His-lle-Gly-Glu) |
| SEQ ID NO:11 | P20 | cyclo(DPhe-Pro-Gln-Ile-Met-Arg-Ile-Lys-Pro-His-Gln-Gly-Gln-His-Ile-Gly) |
| SEQ ID NO :12 | P21 | cycfo(DPhe-Pro-Ile-Met-Arg-Ile-Lys-Pro-His-Gln-Gly-Gln-His-Ile) |
| SEQ ID NO : 24 | P22 | cyclo(Glu-Gln-Ile-Met- Arg-Ile-Lys-Pro-His-Gln) |
| SEQ ID NO :13 | P23 | cyclo(DTyr-Pro-Arg-Ile-Lys-Pro-His-Gln) |
| SEQ ID NO : 25 | P24 | cyclo(Gly-Arg-Ile-Lys-Pro-His) |

**Tableau 2**

| **Peptide** | **Séquence d'acides aminés de VEGF₁₆₅** | **Nombre d'acides aminés** | **Méthode de synthèse** | **Masse Moléculaire** | | **IC₅₀ (µM)** |
|---|---|---|---|---|---|---|
| | | | | calculée | trouvée | |
| P1 | 82-86 | 5 | | 649,8 | 650,3 | >300,0 |
| P2 | 79-86 | 8 | | 1022,3 | 1022,1 | >300,0 |
| P3 | 82-88 | 7 | | 835,0 | 836,6 | >300,0 |
| P4 | 79-93 | 15 | | 1772,1 | 1773,3 | >300,0 |
| P5 | 79-92. | 14 | | 1642,9 | 1644,1 | >300,0 |
| P6 | Gly⁸¹-82-90- DPro⁸⁵ | 10 | | 1157,3 | 1158,4 | >300,0 |
| P7 | (Gly⁸¹.82-90)DPro⁸⁵ | 10 | A | 1139,3 | 1139,9 | >300,0 |
| P8 | (Gly⁸¹-82-88-His⁸⁹) | 9 | A | 1011,2 | 1012,0 | 200,0 |
| P9 | (Pro⁸¹-82-90) | 10 | A | 1179,6 | 1180,6 | 300,0 |
| P10 | 79-93-Oallyle | 15 | | 1821,1 | 1813,1 | >300,0 |
| P11 | (DPhe⁷⁷-Pro⁷⁸-79-93) | 17 | A | 1998,3 | 1998,7 | 2,0 |
| P12 | (Gly⁷⁸-79-93) | 16 | A, B | 1811,1 | 1813,1 | 300,0 |
| P13 | (Pro⁷⁸-79-93) | 16 | A, B | 1851,2 | 1851,4 | >300,0 |
| P14 | Pro⁷⁸-79-93 | 16 | | 1829,1 | 1869,0 | >300,0 |
| P15 | Gly⁷⁸-79-93 | 16 | | 1829,1 | 1828,5 | >300,0 |
| P16 | (82-88) | 7 | A | 817,0 | 817,9 | 32,0 |
| P17 | (Pro⁸¹-82-88) | 8 | A | 914,1 | 915,6 | 10,0 |
| P18 | (DPhe⁸¹--82-87) | 7 | A | 1004,2 | 1004,7 | > 300,0 |
| P19 | (79-93) | 15 | B | 1754.0 | 1755,0 | 5,0 |
| P20 | (DPhe⁷⁷-Pro⁷⁸-79-92) | 16 | B | 1869,2 | 1869,7 | 10,0 |
| P21 | (DPhe⁷⁸-Pro⁷⁹-80-91) | 14 | B | 1684,0 | 1685,4 | 200,0 |
| P22 | (Glu⁷⁸-79-87) | 10 | B | 1261,5 | 1262,7 | >300,0 |
| P23 | (DTyr⁸⁰-Pro⁸¹-82-87) | 8 | A | 1020,2 | 1021,7 | 8,0 |
| P24 | (Gly⁸¹-82-86) | 6 | A | 688,8 | 689,9 | 8,0 |
| | | | | | | |
| | | | | | | |

## Revendications

1. Cyclopeptide choisi parmi les composés suivants :
P11 : cyclo(DPhe-Pro-Gln-Ile-Met-Arg-Ile-Lys-Pro-His-Gln-Gly-Gln-His-Ile-Gly-Glu) SEQ ID NO : 5
P16 : cyclo(Arg-Ile-Lys-Pro-His-Gln-Gly) SEQ ID NO : 8
P17 : cyclo(Pro-Arg-Ile-Lys-Pro-His-Gln-Gly) SEQ ID NO: 9
P19 : cyclo(Gln-Ile-Met-Arg-Ile-Lys-Pro-His-Gln-Gly-Gln-His-Ile-Gly-Glu) SEQ ID NO : 10
P20 : cyclo(DPhe-Pro-Gln-lle-Met-Arg-lle-Lys-Pro-His-Gln-Gly-Gln-His-lle-Gly) SEQ ID NO:11
P23 : cyclo(DTyr-Pro-Arg-lle-Lys-Pro-His-Gln) SEQ ID NO:13
P24 : cyclo(Gly-Arg-lle-Lys-Pro-His) SEQ ID NO: 25.

2. Composition pharmaceutique pour inhiber l'angiogenèse comprenant un cyclopeptide choisi parmi les cyclopeptides :
P11 : cyclo(DPhe-Pro-Gln-Ile-Met-Arg-Ile-Lys-Pro-His-Gln-Gly-Gln-His-Ile-Gly-Glu) SEQ ID NO : 5
P16 : cyclo(Arg-Ile-Lys-Pro-His-Gln-Gly) SEQ ID NO : 8
P17 : cyclo(Pro-Arg-Ile-Lys-Pro-His-Gln-Gly) SEQ ID NO : 9
P19 : cyclo(Gln-Ile-Met-Arg-Ile-Lys-Pro-His-Gln-Gly-Gln-His-Ile-Gly-Glu) SEQ ID NO : 10
P20 : cyclo(DPhe-Pro-Gln-Ile-Met-Arg-Ile-Lys-Pro-His-Gln-Gly-Gln-His-Ile-Gly) SEQ ID NO:11
P23 : cyclo(DTyr-Pro-Arg-Ile-Lys-Pro-His-Gln) SEQ ID NO : 13
P24 : cyclo(Gly-Arg-Ile-Lys-Pro-His) SEQ ID NO: 25.

3. Composition pharmaceutique pour activer l'angiogenèse comprenant deux cyclopeptides identiques ou différents, couplés avec un composé organique pharmaceutiquement acceptable, les cyclopeptides étant choisis parmi les cyclopeptides :
P11 : cyclo(DPhe-Pro-Gln-Ile-Met-Arg-Ile-Lys-Pro-His-Gln-Gly-Gln-His-Ile-Gly-Glu) SEQ ID NO : 5
P16 : cyclo(Arg-Ile-Lys-Pro-His-Gln-Gly) SEQ ID NO : 8
P17 : cyclo(Pro-Arg-Ile-Lys-Pro-His-Gln-Gly) SEQ ID NO : 9
P19 : cyclo(Gln-Ile-Met-Arg-Ile-Lys-Pro-His-Gln-Gly-Gln-His-Ile-Gly-Glu) SEQ ID NO : 10
P20 : cyclo(DPhe-Pro-Gln-Ile-Met-Arg-Ile-Lys-Pro-His-Gln-Gly-Gln-His-Ile-Gly) SEQ ID NO : 11
P23 : cyclo(DTyr-Pro-Arg-Ile-Lys-Pro-His-Gln) SEQ ID NO : 13
P24 : cyclo(Gly-Arg-Ile-Lys-Pro-His) SEQ ID NO : 25.

4. Système comportant un cyclopeptide selon la revendication 1, lié de façon covalente à un bras espaceur organique.

5. Système selon la revendication 4, dans lequel le bras espaceur organique est lié de façon covalente à un support.

6. Système comportant deux cyclopeptides selon la revendication 1, lié de façon covalente à un composé organique.

7. Système selon la revendication 6, dans lequel la distance entre les deux cyclopeptides est telle que, lorsque le système est mis en contact avec des cellules exprimant les récepteurs du facteur de croissance de l'endothélium vasculaire (VEGF), il autorise la dimérisation de ces récepteurs.

8. Système selon l'une quelconque des revendications 6 et 7, dans lequel le composé organique est lié de façon covalente à un support par l'intermédiaire d'un bras espaceur organique.

9. Système comportant un support solide sur lequel sont fixés par liaison covalente des cyclopeptides selon la revendication 1 chacun des cyclopeptides étant relié au support par un bras espaceur organique.

10. Système selon l'une quelconque des revendications 4, 5, 8 et 9 ; dans lequel le bras espaceur comprend une chaîne hydrocarbonée, fluorocarbonée, polyéther, polyéthylène glycol, polyamine, polyamide, polyester, polysiloxane ou une combinaison de celles-ci, qui est fonctionnalisée à chaque extrémité pour former une liaison ,covalente, d'une part, avec le cyclopeptide et, d'autre part, avec le support.

11. Système selon l'une quelconque des revendications 4 à 10, dans lequel le bras espaceur organique comprend de plus un motif susceptible d'être coupé par un système enzymatique.

12. Système selon la revendication 11, dans lequel le bras espaceur organique comprend de plus un composé bioactif.

13. Système selon l'une quelconque des revendications 5, 8 et 9, dans lequel le support est un solide organique ou inorganique.

14. Système selon l'une quelconque des revendications 5, 8 et 9, dans lequel le support est un polymère organique sous forme solide ou sous forme de gel.

15. Système selon la revendication 14, dans lequel le polymère organique est un polymère biocompatible, biodégradable ou non.

16. Système selon la revendication 15, dans lequel le polymère organique est choisi parmi le polytéréphtalate d'éthylène, les copolymères de fluorure de vinylidène et d'hexafluoropropylène, les alcools polyvinyliques, les polyhydroxyéthyl méthacrylate, les polysacharides et leurs copolymères.

17. Système selon l'une quelconque des revendications 4 et 5 pour inhiber l'angiogenèse.

18. système selon l'une quelconque des revendications 6 à 8 pour activer l'angiogenèse.

19. Procédé de préparation d'un système selon l'une quelconque des revendications 14 à 16, qui consiste à soumettre un support en polymère organique à une irradiation au moyen de rayonnements ionisants, de plasmas ou de photons, sur des zones définies du support, et à greffer ensuite sur ces zones du support le bras espaceur organique.

20. Procédé selon la revendication 19, dans lequel on réalise l'irradiation au travers d'un masque.

21. Procédé selon la revendication 19, dans lequel les rayonnements ionisants sont un faisceau d'électrons ou d'ions lourds rapides.

22. Procédé selon la revendication 19, dans lequel les cyclopeptides sont fixés sur les bras espaceurs organiques, avant greffage.

23. Procédé selon la revendication 19, qui comprend de plus une étape de fixation des cyclopeptides sur les bras espaceurs organiques, après greffage de ceux-ci.

## Claims

1. Cyclopeptide chosen from among the following compounds :
P11: cyclo(DPhe-Pro-Gln-Ile-Met-Arg-Ile-Lys-Pro-His-Gln-Gly-Gln-His-Ile-Gly-Glu) SEQ ID NO:5
P16: cyclo(Arg-Ile-Lys-Pro-His-Gln-Gly) SEQ ID NO:8
P17: cyclo(Pro-Arg-Ile-Lys-Pro-His-Gln-Gly) SEQ ID NO:9
P19: cyclo(Gln-Ile-Met-Arg-Ile-Lys-Pro-His-Gln-Gly-Gln-His-Ile-Gly-Glu) SEQ ID NO:10
P20: cyclo(DPhe-Pro-Gln-Ile-Met-Arg-Ile-Lys-Pro-His-Gln-Gly-Gln-His-Ile-Gly) SEQ ID NO:11
P23: cyclo(DTyr-Pro-Arg-Ile-Lys-Pro-His-Gln) SEQ ID NO:13
P24: cyclo(Gly-Arg-Ile-Lys-Pro-His) SEQ ID NO:25.

2. Pharmaceutical composition for inhibiting angiogenesis comprising a cyclopeptide chosen among the cyclopeptides:
P11: cyclo(DPhe-Pro-Gln-Ile-Met-Arg-Ile-Lys-Pro-His-Gln-Gly-Gln-His-Ile-Gly-Glu) SEQ ID NO:5
P16: cyclo (Arg-Ile-Lys-Pro-His-Gln-Gly) SEQ ID NO:8
P17: cyclo(Pro-Arg-Ile-Lys-Pro-His-Gln-Gly) SEQ ID NO:9
P19: cyclo(Gln-Ile-Met-Arg-Ile-Lys-Pro-His-Gln-Gly-Gln-His-Ile-Gly-Glu) SEQ ID NO:10
P20: cyclo(DPhe-Pro-Gln-lle-Met-Arg-lle-Lys-Pro-His-Gln-Gly-Gln-His-lle-Gly) SEQ ID NO:11
P23: cyclo(DTyr-Pro-Arg-Ile-Lys-Pro-His-Gln) SEQ ID NO:13
P24: cyclo(Gly-Arg-Ile-Lys-Pro-His) SEQ ID NO:25.

3. Pharmaceutical composition for activating angiogenesis comprising two identical or different cyclopeptides, coupled with a pharmaceutically acceptable organic compound, the cyclopeptides being chosen from among the cyclopeptides:
P11: cyclo(DPhe-Pro-Gln-Ile-Met-Arg-Ile-Lys-Pro-His-Gln-Gly-Gln-His-Ile-Gly-Glu) SEQ ID NO:5
P16: cyclo(Arg-Ile-Lys-Prc-His-Gln-Gly; SEQ ID NO:8
P17: cyclo(Pro-Arg-Ile-Lys-Pro-His-Gln-Gly) SEQ ID NO:9
P19: cyclo(Gln-Ile-Met-Arg-Ile-Lys-Pro-His-Gln-Gly-Gln-His-Ile-Gly-Glu) SEQ ID NO:10
P20: cyclo(DPhe-Pro-Gln-Ile-Met-Arg-Ile-Lys-Pro-His-Gln-Gly-Gln-His-Ile-Gly) SEQ ID NO:11
P23: cyclo(DTyr-Pro-Arg-Ile-Lys-Pro-His-Gln) SEQ ID NO:13
P24: cyclo(Gly-Arg-Ile-Lys-Pro-His) SEQ ID NO:25.

4. System comprising a cyclopeptide according to claim 1, covalently bound to an organic spacer arm.

5. System according to claim 4, wherein the organic spacer arm is covalently bound to a support.

6. System comprising two cyclopeptides according to claim 1, covalently bound to an organic compound.

7. System according to claim 6, wherein the distance between the two cyclopeptides is such that, when the system is placed in contact with cells expressing vascular endothelium growth factor (VEGF) receptors, it allows dimerization of said receptors.

8. System according to any one of claims 6 and 7, wherein the organic compound is covalently bound to a support with the aid of an organic spacer arm.

9. System comprising a solid support upon which cyclopeptides according to claim 1 are fixed by covalent bonding, each of the cyclopeptides being bound to the support by an organic spacer arm.

10. System according to any one of claims 4, 5, 8 and 9, wherein the spacer arm comprises a hydrocarbon, fluorocarbon, polyether, polyethylene glycol, polyamine, polyamide, polyester, polysiloxane chain or a combination thereof that is functionalized at each end for forming a covalent bond on the one hand with the cyclopeptide and on the other hand with the support.

11. System according to any one of claims 4 to 10, wherein the organic spacer arm further comprises a moiety capable of being cut by an enzymatic system.

12. System according to claim 11, wherein the organic spacer arm further comprises a bioactive compound.

13. System according to any one of claims 5, 8 and 9, wherein the support is an organic or an inorganic solid.

14. System according to any one of claims 5, 8 and 9, wherein the support is an organic polymer in solid form or gel form.

15. System according to claim 14, wherein the organic polymer is a biocompatible, biodegradable or non-biodegradable polymer.

16. System according to claim 15, wherein the organic polymer is chosen from among ethylene polyterephthalate, the copolymers of vinylidene fluoride and hexafluoropropylene, the polyvinyl alcohols, the polyhydroxyethyl methacrylates, the polysaccharides and their copolymers.

17. System according to any one of claims 4 and 5 for inhibiting angiogenesis.

18. System according to any one of claims 6 to 8 for activating angiogenesis.

19. Method of preparing a system according to any one of claims 14 to 16, which consists in subjecting a support made of organic polymer to an irradiation by means of ionizing radiations, plasmas, or photons onto defined zones of the support and then grafting the organic spacer arm onto these zones of the support.

20. Method according to claim 19, wherein the irradiation is carried out through a mask.

21. Method according to claim 19, wherein the ionizing radiations are a beam of electrons or fast heavy ions.

22. Method according to claim 19, wherein the cyclopeptides are fixed on the organic spacer arms prior to grafting.

23. Method according to claim 19, which further comprises a step of fixation of the cyclopeptides on the organic spacer arms after grafting of same.

## Patentansprüche

1. Cyclopeptid, ausgewählt aus den folgenden Verbindungen:
P11: Cyclo(DPhe-Pro-Gln-Ile-Met-Arg-Ile-Lys-Pro-His-Gln-Gly-Gln-His-Ile-Gly-Glu) SEQ ID Nr. 5
P16: Cyclo(Arg-Ile-Lys-Pro-His-Gln-Gly) SEQ ID Nr. 8
P17: Cyclo(Pro-Arg-Ile-Lys-Pro-His-Gln-Gly) SEQ ID Nr. 9
P19: Cyclo(Gln-Ile-Met-Arg-Ile-Lys-Pro-His-Gln-Gly-Gln-His-Ile-Gly-Glu) SEQ ID Nr. 10
P20: Cyclo(DPhe-Pro-Gln-Ile-Met-Arg-Ile-Lys-Pro-His-Gln-Gly-Gln-His-Ile-Gly) SEQ ID Nr. 11
P23: Cyclo (DTyr-Pro-Arg-Ile-Lys-Pro-His-Gln) SEQ ID Nr. 13
P24 : Cyclo(Gly-Arg-Ile-Lys-Pro-His) SEQ ID Nr. 25.

2. Pharmazeutische Zusammensetzung zum Hemmen (Verhindern) der Angiogenese, die ein Cyclopeptid umfasst, das ausgewählt ist aus den folgenden Cyclopeptiden:
P11: Cyclo(DPhe-Pro-Gln-Ile-Met-Arg-Ile-Lys-Pro-His-Gln-Gly-Gln-His-Ile-Gly-Glu) SEQ ID Nr: 5
P16: Cyclo(Arg-Ile-Lys-Pro-His-Gln-Gly) SEQ ID Nr: 8
P17: Cyclo(Pro-Arg-Ile-Lys-Pro-His-Gln-Gly) SEQ ID Nr: 9
P19: Cyclo(Gln-Ile-Met-Arg-Ile-Lys-Pro-His-Gln-Gly-Gln-His-Ile-Gly-Glu) SEQ ID Nr: 10
P20: Cyclo(DPhe-Pro-Gln-lle-Met-Arg-lle-Lys-Pro-His-Gln-Gly-Gln-His-lle-Gly) SEQ ID Nr. 11
P23: Cyclo(DTyr-Pro-Arg-Ile-Lys-Pro-His-Gln) SEQ ID Nr. 13
P24: Cyclo(Gly-Arg-Ile-Lys-Pro-His) SEQ ID Nr. 25.

3. Pharmazeutische Zusammensetzung zum Aktivieren der Angiogenese, die zwei identische oder unterschiedliche Cyclopeptide umfasst, die mit einer pharmazeutisch akzeptablen organischen Verbindung gekoppelt sind, wobei die Cyclopeptide ausgewählt sind aus den folgenden Cyclopeptiden:
P11: Cyclo(DPhe-Pro-Gln-Ile-Met-Arg-Ile-Lys-Pro-His-Gln-Gly-Gln-His-Ile-Gly-Glu) SEQ ID Nr. 5
P16: Cyclo(Arg-Ile-Lys-Pro-His-Gln-Gly) SEQ ID Nr. 8
P17: Cyclo(Pro-Arg-Ile-Lys-Pro-His-Gln-Gly) SEQ ID Nr. 9
P19: Cyclo(Gln-Ile-Met-Arg-Ile-Lys-Pro-His-Gln-Gly-Gln-His-Ile-Gly-Glu) SEQ ID Nr. 10
P20: Cyclo(DPhe-Pro-Gln-Ile-Met-Arg-Ile-Lys-Pro-His-Gln-Gly-Gln-His-Ile-Gly) SEQ ID Nr. 11
P23: Cyclo(DTyr-Pro-Arg-Ile-Lys-Pro-His-Gln) SEQ ID Nr. 13
P24 : Cyclo(Gly-Arg-Ile-Lys-Pro-His) SEQ ID Nr. 25.

4. System, das ein Cyclopeptid nach Anspruch 1 umfasst, das an einen organischen Abstandhalter-Arm kovalent gebunden ist.

5. System nach Anspruch 4, in dem der organische Abstandhalter-Arm kovalent an einen Träger gebunden ist.

6. System, das zwei Cyclopeptide nach Anspruch 1 umfasst, die an eine organische Verbindung kovalent gebunden sind.

7. System nach Anspruch 6, in dem der Abstand zwischen den beiden Cyclopeptiden derart ist, dass dann, wenn das System mit Zellen in Kontakt gebracht wird, welche die Rezeptoren des Wachstumsfaktors des Gefäßendothels (VEGF) exprimieren, der Abstand die Dimerisation dieser Rezeptoren ermöglicht.

8. System nach einem der Ansprüche 6 und 7, in dem die organische Verbindung mittels eines organischen Abstandhalter-Arms kovalent an einen Träger gebunden ist.

9. System, das einen festen Träger umfasst, an dem Cyclopeptide nach Anspruch 1 durch eine kovalente Bindung fixiert sind, wobei jedes der Cyclopeptide mittels eines organischen Abstandhalter-Arms an den Träger gebunden ist.

10. System nach einem der Ansprüche 4, 5, 8 und 9, in dem der Abstandhalter-Arm eine Kohlenwasserstoff-, Fluorkohlenstoff-, Polyether-, Polyethylenglycol-, Polyamin-, Polyamid-, Polyester-, Polysiloxan-Kette oder eine Kombination derselben umfasst, die an jedem Ende funktionalisiert ist (eine funktionelle Gruppe aufweist), um eine kovalente Bindung auszubilden einerseits gegenüber dem Cyclopeptid und andererseits gegenüber dem Träger.

11. System nach einem der Ansprüche 4 bis 10, in dem der organische Abstandhalter-Arm außerdem eine wiederkehrende Einheit umfasst, die durch ein enzymatisches System geschnitten werden kann.

12. System nach Anspruch 11, in dem der organische Abstandhalter-Arm außerdem eine bioaktive Verbindung umfasst.

13. System nach einem der Ansprüche 5, 8 und 9, in dem der Träger ein organischer oder anorganischer Feststoff ist.

14. System nach einem der Ansprüche 5, 8 und 9, in dem der Träger ein organisches Polymer in Form eines Feststoffes oder in Form eines Gels ist.

15. System nach Anspruch 14, in dem das organische Polymer ein biokompatibles, biologisch abbaubares oder nicht abbaubares Polymer ist.

16. System nach Anspruch 15, in dem das organische Polymer ausgewählt ist aus der Gruppe Ethylenpolyterephthalat, der Copolymeren von Vinylidenfluorid und Hexafluorpropylen, der Polyvinylalkohole, der Polyhydroxyethylmethacrylate, der Polysacharide und ihren Copolymeren.

17. System nach einem der Ansprüche 4 und 5 zum Hemmen (Verhindern) der Angiogenese.

18. System nach einem der Ansprüche 6 bis 8 zum Aktivieren der Angiogenese.

19. Verfahren zur Herstellung eines Systems nach einem der Ansprüche 14 bis 16, das darin besteht, dass man einen Träger aus einem organischen Polymer auf definierten Zonen des Trägers mit ionisierenden Strahlen, Plasmastrahlen oder Photonenstrahlen bestrahlt und anschließend auf diese Zonen des Trägers den organischen Abstandhalter-Arm aufpfropft.

20. Verfahren nach Anspruch 19, bei dem man die Bestrahlung durch eine Maske hindurch durchführt.

21. Vorfahren nach Anspruch 19, bei dem die ionisierenden Strahlen ein Bündel von Elektronen oder schnellen schweren Ionen darstellen.

22. Verfahren nach Anspruch 19, bei dem die Cyclopeptide vor dem Aufpfropfen an den organischen Abstandhalter-Armen fixiert werden.

23. Verfahren nach Anspruch 19, das außerdem eine Stufe umfasst, in der die Polypeptide an den organischen Abstandhalter-Armen fixiert werden, nachdem diese aufgepfropft worden sind.


### LISTE DE SEQUENCES

<110> Commissariat à l'énergie atomique
   Université Victor Segalen - Bordeaux 2
   Université Bordeaux 1
   BETZ Natacha
   BIKFALVI Andréas
   DELERIS Gérard
<120> Cyclopeptides, leur procédé de préparation et leur utilisation comme inhibiteur ou activateur de l'angiogenèse
<130> B 13445.3 MDT
<140>
   <141>
<150> FR n° 0012654
   <151> 2000-10-04
<160> 25
<170> PatentIn Ver. 2.1
<210> 1
   <211> 7
   <212> PRT
   <213> Séquence artificielle
<220>
   <223> Description de la séquence artificielle: séquence issue de VEGF-A
<400> 1
   Arg Ile Lys Pro His Gln Gly
   1 5
<210> 2
   <211> 10
   <212> PRT
   <213> Séquence artificielle
<220>
   <223> Description de la séquence artificielle: séquence issue de VEGF-A
<220>
   <223> peptide cyclique
<400> 2
   Gly Arg Ile Lys Pro His Gln Gly Gln His
   1 5 10
<210> 3
   <211> 9
   <212> PRT
   <213> Séquence artificielle
<220>
   <223> Description de la séquence artificielle: séquence issue de VEGF-A
<220>
   <223> peptide cyclique
<220>
   <221> MOD_RES
   <222> (5)
   <223> (D) Pro
<400> 3
   Gly Arg Ile Lys Pro His Gln Gly His
   1 5
<210> 4
   <211> 10
   <212> PRT
   <213> Séquence artificielle
<220>
   <223> Description de la séquence artificielle: séquence issue de VEGF-A
<220>
   <223> peptide cyclique
<400> 4
   Pro Arg Ile Lys Pro His Gln Gly Gln His
    1               5                   10
<210> 5
   <211> 17
   <212> PRT
   <213> Séquence artificielle
<220>
   <223> Description de la séquence artificielle: séquence issue de VEGF-A
<220>
   <223> peptide cyclique
<220>
   <221> MOD_RES
   <222> (1)
   <223> (D)Phe
<400> 5
   Phe Pro Gln Ile Met Arg Ile Lys Pro His Gln Gly Gln His Ile Gly
    1               5                   10                  15
   Glu
<210> 6
   <211> 16
<212> PRT
   <213> Séquence artificielle
<220>
   <223> Description de la séquence artificielle: séquence issue de VEGF-A
<220>
   <223> peptide cyclique
<400> 6
   Gly Gln Ile Met Arg Ile Lys Pro His Gln Gly Gln His Ile Gly Glu
    1               5                   10                  15
<210> 7
   <211> 16
   <212> PRT
   <213> Séquence artificielle
<220>
   <223> Description de la séquence artificielle: séquence issue de VEGF-A
<220>
   <223> peptide cyclique
<400> 7
   Pro Gln Ile Met Arg Ile Lys Pro His Gln Gly Gln His Ile Gly Glu
    1               5                  10                  15
<210> 8
   <211> 7
   <212> PRT
   <213> Séquence artificielle
<220>
   <223> Description de la séquence artificielle: séquence issue de VEGF-A
<220>
   <223> peptide cyclique
<400> 8
   Arg Ile Lys Pro His Gln Gly
    1               5
<210> 9
   <211> 8
   <212> PRT
   <213> Séquence artificielle
<220>
   <223> Description de la séquence artificielle: séquence issue de VEGF-A
<220>
   <223> peptide cyclique
<400> 9
   Pro Arg Ile Lys Pro His Gln Gly
    1               5
<210> 10
   <211> 15
   <212> PRT
   <213> Séquence artificielle
<220>
   <223> Description de la séquence artificielle: séquence issue de VEGF-A
<220>
   <223> peptide cyclique
<400> 10
   Gln Ile Met Arg Ile Lys Pro His Gln Gly Gln His Ile Gly Glu
    1               5                   10                  15
<210> 11
   <211> 16
   <212> PRT
   <213> Séquence artificielle
<220>
   <223> Description de la séquence artificielle: séquence issue de VEGF-A
<220>
   <223> peptide cyclique
<220>
   <221> MOD_RES
   <222> (1)
   <223> (D)Phe
<400> 11
   Phe Pro Gln Ile Met Arg Ile Lys Pro His Gln Gly Gln His Ile Gly
    1               5                   10                  15
<210> 12
   <211> 14
   <212> PRT
   <213> Séquence artificielle
<220>
   <223> Description de la séquence artificielle: séquence issue de VEGF-A
<220>
   <223> peptide cyclique
<220>
   <221> MOD_RES
   <222> (1)
   <223> (D)Phe
<400> 12
   Phe Pro Ile Met Arg Ile Lys Pro His Gln Gly Gln His Ile
    1               5                   10
<210> 13
   <211> 8
   <212> PRT
   <213> Séquence artificielle
<220>
   <223> Description de la séquence artificielle: séquence issue de VEGF-A
<220>
   <223> peptide cyclique
<220>
   <221> MOD_RES
   <222> (1)
   <223> (D)Tyr
<400> 13
   Tyr Pro Arg Ile Lys Pro His Gln
    1               5
<210> 14
   <211> 5
   <212> PRT
   <213> Séquence artificielle
<220>
   <223> Description de la séquence artificielle: séquence issue de VEGF-A
<400> 14
   Arg Ile Lys Pro His
    1               5
<210> 15
   <211> 8
   <212> PRT
   <213> Séquence artificielle
<220>
   <223> Description de la séquence artificielle: séquence issue de VEGF-A
<400> 15
   Gln Ile Met Arg Ile Lys Pro His
    1               5
<210> 16
   <211> 7
   <212> PRT
   <213> Séquence artificielle
<220>
   <223> Description de la séquence artificielle: séquence issue de VEGF-A
<400> 16
   Arg Ile Lys Pro His Gln Gly
   1 5
<210> 17
   <211> 15
   <212> PRT
   <213> Séquence artificielle
<220>
   <223> Description de la séquence artificielle: séquence issue de VEGF-A
<400> 17
   Gln Ile Met Arg Ile Lys Pro His Gln Gly Gln His Ile Gly Glu
   1 5 10 15
<210> 18
   <211> 14
   <212> PRT
   <213> Séquence artificielle
<220>
   <223> Description de la séquence artificielle: séquence issue de VEGF-A
<400> 18
   Gln Ile Met Arg Ile Lys Pro His Gln Gly Gln His Ile Gly
   1 5 10
<210> 19
   <211> 10
   <212> PRT
   <213> Séquence artificielle
<220>
   <223> Description de la séquence artificielle: séquence issue de VEGF-A
<220>
   <221> MOD_RES
   <222> (5)
   <223> (D)Pro
<400> 19
   Gly Arg Ile Lys Pro His Gln Gly Gln His
   1 5 10
<210> 20
   <211> 15
   <212> PRT
   <213> Séquence artificielle
<220>
   <223> Description de la séquence artificielle: séquence issue de VEGF-A
<400> 20
   Gln Ile Met Arg Ile Lys Pro His Gln Gly Gln His Ile Gly Glu
   1 5 10 15
<210> 21
   <211> 16
   <212> PRT
   <213> Séquence artificielle
<220>
   <223> Description de la séquence artificielle: séquence issue de VEGF-A
<400> 21
   Pro Gln Ile Met Arg Ile Lys Pro His Gln Gly Gln His Ile Gly Glu
   1 5 10 15
<210> 22
   <211> 16
   <212> PRT
   <213> Séquence artificielle
<220>
   <223> Description de la séquence artificielle: séquence issue de VEGF-A
<400> 22
   Gly Gln Ile Met Arg Ile Lys Pro His Gln Gly Gln His Ile Gly Glu
   1 5 10 15
<210> 23
   <211> 7
   <212> PRT
   <213> Séquence artificielle
<220>
   <223> Description de la séquence artificielle: séquence issue de VEGF-A
<220>
   <223> peptide cyclique
<220>
   <221> MOD_RES
   <222> (1)
   <223> (D)Phe
<400> 23
   Phe Arg Ile Lys Pro His Gln
   1 5
<210> 24
   <211> 10
   <212> PRT
   <213> Séquence artificielle
<220>
   <223> Description de la séquence artificielle: séquence issue de VEGF-A
<220>
   <223> peptide cyclique
<400> 24
   Glu Gln Ile Met Arg Ile Lys Pro His Gln
   1 5 10
<210> 25
   <211> 6
   <212> PRT
   <213> Séquence artificielle
<220>
   <223> Description de la séquence artificielle: séquence issue de VEGF-A
<220>
   <223> peptide cyclique
<400> 25
   Gly Arg Ile Lys Pro His
   1 5